# EUROPEAN PATENT SPECIFICATION

(11) **EP 4 072 672 B1**
(45) Date of publication and mention of the grant of the patent: **02.09.2026**
(21) Application number: 20839171.4
(22) Date of filing: 10.12.2020
(51) Int. Cl.: C07K 16/40, A61K 39/395, A61P 3/06, A61P 9/06

(54) **USE OF ALIROCUMAB TO TREAT HOMOZYGOUS FAMILIAL HYPERCHOLESTEROLEMIA**
VERWENDUNG VON ALIROCUMAB ZUR BEHANDLUNG VON HOMOZYGOTER FAMILIÄRER HYPERCHOLESTERINÄMIE
UTILISATION DE ALIROCUMAB POUR TRAITER L'HYPERCHOLESTÉROLÉMIE FAMILIALE HOMOZYGOTE

(30) Priority: 10.12.2019 US 201962946268 P; 09.03.2020 US 202062987148 P
(43) Date of publication of application: 19.10.2022
(73) Proprietor: Regeneron Pharmaceuticals, Inc., Tarrytown, NY 10591 (US); Sanofi Biotechnology, 75008 Paris (FR)
(72) Inventor: ALI, Shazia, Tarrytown, New York 10591 (US); BACCARA-DINET, Marie, 75008 Paris (FR); DONAHUE, Stephen, Tarrytown, New York 10591 (US); HANOTIN, Corinne, 75008 Paris (FR); LECORPS, Guillaume, 75008 Paris (FR); PORDY, Robert C., Tarrytown, New York 10591 (US)
(74) Representative: Zwicker, Jörk
(86) International application number: PCT/US2020/064324
(87) International publication number: WO 2021/119321

(56) References cited:
- WO-A1-2013/039969
- WO-A1-2014/194111
- WO-A1-2016/011256
- WO-A2-2013/016648
- WO-A2-2015/054619
- PT-T- 3 689 913
- US-A1- 2010 166 768
- US-A1- 2014 356 371
- US-A1- 2015 004 174
- LAMBERT GILLES ET AL: "Normalization of Low-Density Lipoprotein Receptor Expression in Receptor Defective Homozygous Familial Hypercholesterolemia by Inhibition of PCSK9 With Alirocumab", JOURNAL OF THE AMERICAN COLLEGE OF CARDIOLOGY, vol. 64, no. 21, 1 December 2014 (2014-12-01), NEW YORK, NY, pages 2299 - 2300, XP055791056, ISSN: 0735-1097, DOI: 10.1016/j.jacc.2014.07.995
- LOK-YI CHAN M ET AL: "Response to alirocumab in homozygous familial hypercholesterolemia: A case report", 18TH INTERNATIONAL SYMPOSIUM ON ATHEROSCLEROSIS, ISA 2018, 9 June 2018 (2018-06-09), pages 43 - 44, XP055791131, Retrieved from the Internet <URL:https://www.sciencedirect.com/science/article/pii/S1567568818301296> [retrieved on 20210329]
- RAAL FREDERICK J ET AL: "Inhibition of PCSK9 with evolocumab in homozygous familial hypercholesterolaemia (TESLA Part B): a randomised, double-blind, placebo-controlled trial", THE LANCET, vol. 385, no. 9965, 1 January 2015 (2015-01-01), AMSTERDAM, NL, pages 341 - 350, XP055791108, ISSN: 0140-6736, DOI: 10.1016/S0140-6736(14)61374-X
- NAJAM O ET AL: "The past, present and future of lipid-lowering therapy", CLINICAL LIPIDOLOGY, FUTURE MEDICINE LTD, GB, vol. 10, no. 6, 1 December 2015 (2015-12-01), pages 481 - 498, XP009501101, ISSN: 1758-4299, DOI: 10.2217/CLP.15.40
- IOANNA GOUNI-BERTHOLD ET AL: "PCSK9 Antibodies for the Treatment of Hypercholesterolemia", NUTRIENTS, vol. 6, no. 12, 1 January 2014 (2014-01-01), pages 5517 - 5533, XP055312148, DOI: 10.3390/nu6125517

## Description

### FIELD

The present disclosure relates to a PCSK9 inhibitor for use in treating patients with homozygous familial hypercholesterolemia (hoFH) who are refractory to statin treatment, who are intolerant to statins, or who otherwise have a history of adverse reactions to statin therapy.

### BACKGROUND

Familial hypercholesterolemia (FH) is an inherited disorder of lipid metabolism that predisposes a person to premature severe cardiovascular disease (CVD) (Kolansky, et al. 2008 Am J Cardiol 102(11):1438-1443). It can be either an autosomal dominant or an autosomal recessive disease that results from mutations in the low density lipoprotein receptor (LDLR), or in 3 associated genes: proprotein convertase subtilisin/kexin type 9 (PCSK9), apolipoprotein B (Apo B), and LDL receptor adaptor protein 1 (LDLRAP1), with a similar phenotype and varying severity.

Homozygous familial hypercholesterolemia (hoFH) is a rare, serious condition genetically defined to include individuals with the same mutation(s) in both LDLR, ApoB, or PCSK9 alleles (true homozygotes), different mutations in each allele of the same gene (compound heterozygotes), or different mutations on different genes (double heterozygotes). Phenotypically, the severity of hoFH depends on the amount of residual LDLR activity, historically categorized as either receptor-negative (<2% of normal LDLR activity) or receptor-defective (2% to 25% of normal LDLR activity) based on the amount of activity in skin fibroblasts. The genetic definition used herein includes all individuals considered to be true homozygotes, compound heterozygotes, or double heterozygotes. However, those individuals with null LDLR mutations in both alleles are excluded.

Patients with hoFH generally have severe hypercholesterolemia (500-1000 mg/dL, 12.95-25.9 mmol/L), resulting in lifelong exposure to high levels of plasma LDL-C and increased risk of developing atherosclerosis at a highly accelerated rate, often manifesting within the first 2 decades of life. Persistently high levels of LDL-C can also lead to cutaneous and tendon xanthomas, valvular and supravalvular stenosis (Kolansky, et al. 2008 Am J Cardiol 102(11):1438-1443). This accelerated atherosclerosis results in premature cardiovascular disease (CVD) and an increased risk of a cardiovascular (CV) event. A recent observational study of hoFH patients demonstrated that the mean age for first major CV event was 20 years (Kolansky 2008).

The initial goal of drug therapy in adult patients with FH is to achieve LDL-C reduction >_50% (Goldberg, et al. 2011 J Clin Lipidol 5(3 Supp):S1-S8). If this is achieved, therapy is escalated with an aim to achieve an LDL-C of <100 mg/dL (2.59 mmol/L) in the absence of coronary artery disease or other major risk factors or <70 mg/dL (<1.81 mmol/L) in the presence of coronary artery disease or other major risk factors (Watts, et al. 2014 J Clin Lipidol 8(2):148-172). However, management of elevated LDL-C in patients with hoFH is challenging with the current existing treatment options. Patients with hoFH generally have a poor response to conventional drug therapies, resulting in extremely elevated LDL-C levels that are often refractory to pharmacologic management thus requiring the need to initiate LDL apheresis.

Statins inhibit cholesterol synthesis by inhibiting 3-hydroxy-3-methyl-glutaryl coenzyme reductase and are used as first-line therapy in hoFH patients. Patients with hoFH tend to be refractory to statins because the mechanism of action generally lowers LDL-C levels through up-regulation of the hepatic LDL receptor. Nonetheless, despite the near total loss of functional LDL receptors in hoFH patients, statins are still used as first line therapy in order to maximize residual receptor activity (Raal, et al. 2000 Atherosclerosis 150(2):421-428, Marais, et al. 2008 Atherosclerosis 197(1):400-406, Raal, et al. 1997 Atherosclerosis 135(2):249-256).

Many patients treated with the combination of high dose statin and ezetimibe, a cholesterol absorption inhibitor, remain far from their target LDL-C. Newer therapies, *i.e*., mipomersen and lomitapide, have been approved for use in patients with hoFH, but they are not commercially available in all countries and are associated with increases in hepatic fat content, elevated markers of liver injury, frequent injection site reactions that can be of severe intensity (mipomersen) or poorly tolerated gastrointestinal adverse effects (lomitapide) (Raal, et al. 2010 Lancet 375(9719):998-1006, Cuchel, et al. 2013 Lancet 381(9860):40-46).

Mechanical removal of LDL-C using LDL apheresis is an option, but may lower the quality of life in patients and present other challenges (Schiel, et al. 1995 Int J Artif Organs 18(12):786-793). Low-density lipoprotein apheresis is a costly procedure that is invasive and burdensome for patients. US 2014/356371 discloses methods for reducing various lipoprotein fractions in the serum of patients comprising administering a pharmaceutical composition comprising a PCSK9 inhibitor such as antibody mAb316P. WO 2015/054619 provides methods for treating hyperlipidemia in patients who are not on statin therapy. The methods comprise administering to the patient a pharmaceutical composition comprising a PCSK9 inhibitor such as antibody mAb316P. Lambert et al. (J. Am. Col. Cardiology 2017, 64(21)) report normalization of low-density lipoprotein receptor expression in receptor defective homozygous familial hypercholesterolemia by inhibition of PCSK9 with alirocumab. Lok-Yi Chan et al. (18th International Symposium on Atherosclerosis, ISA 2018, pages 43-44) report a patient diagnosed clinically to have severe heterozygous familial hypercholesterolemia who responded to alirocumab. US 2015/004174 discloses methods for treating homozygous familial hypercholesterolemia using antibodies against PCSK9. Raal et al. (The Lancet 2014, 385(9965): 341-350) report inhibition of PCSK9 with evolocumab in homozygous familial hypercholesterolemia in the randomized, double blind, placebo-controlled TESLA study.

### BRIEF SUMMARY

There remains a need for effectively treating patients having homozygous familial hypercholesterolemia (hoFH). Specifically, there remains a need for effectively treating patients having hoFH who are unable to achieve acceptable LDL-C levels despite treatment with statins, do not tolerate or experience adverse reactions to statin therapy, and/or must resort to LDL apheresis for treatment.

The invention is defined by the appended claims. Any reference to methods of treatment refers to the antibodies or pharmaceutical compositions of the present invention for use in a method of treatment of the human body by therapy. The present disclosure provides a human antibody that specifically binds human proprotein convertase subtilisin/kexin type 9 (hPCSK9) for use in treating homozygous familial hypercholesterolemia (hoFH). In particular, the present invention is useful for treating patients with hoFH, excluding those patients with null/null mutations in both LDLR alleles.

In one aspect, the invention provides the antibody comprising a heavy chain comprising SEQ ID NO:5 and a light chain comprising SEQ ID NO:9, a heavy chain variable region (HCVR) comprising SEQ ID NO:1 and a light chain variable domain (LCVR) comprising SEQ ID NO:6, wherein the three heavy chain CDRs are set forth in SEQ ID NOs: 2, 3 and 4, and the three light chain CDRs are set forth in SEQ ID NOs: 7, 8 and 10 for use in treating homozygous familial hypercholesterolemia (hoFH) in a patient in need thereof, wherein the patient having hoFH is refractory to treatment with statins, is intolerant to statins, or has a history of adverse reactions to statin therapy; and wherein the antibody or antigen binding fragment thereof is administered subcutaneously to the patient at a dose of about 150 mg at a frequency of once every two weeks.

In one embodiment, the patient is receiving at least one lipid-modifying therapy (LMT) prior to or at the time of administration of the PCSK9 inhibitor. In further embodiments, the at least one LMT is at least one statin. In still further embodiments, the at least one LMT is LDL apheresis. In still further embodiments, the at least one LMT is ezetimibe.

In one embodiment, the patient has an increased risk for premature cardiovascular disease and/or for a cardiovascular event.

In one embodiment, the antibody is contained in a pre-filled pen delivery device.

In one embodiment, the patient with hoFH has an LDL receptor genotype selected from the group consisting of: (a) homozygous non-null/non-null; (b) compound heterozygous non-null-/non-null; (c) compound heterozygous non-null/null; and (d) homozygous null/null. In another embodiment, the patient with hoFH has an LDL receptor genotype selected from the group consisting of: (a) homozygous non-null/non-null; (b) compound heterozygous non-null-/non-null; and (c) compound heterozygous non-null/null.

In one embodiment, about 12 weeks after administration of the one or more doses of the PCSK9 inhibitor, the patient exhibits one or more lipid parameter improvements selected from the group consisting of:
i) a reduction in LDL-C level from baseline of about 35%;
ii) a reduction in non-HDL-C level from baseline of about 33%;
iii) a reduction in Apo B level from baseline of about 30%;
iv) a reduction in total cholesterol level from baseline of about 27%;
v) a reduction in (fasting) triglyceride level from baseline of about 11%; and/or
vi) a reduction in Lp(a) level from baseline of about 28%.

In one aspect, the disclosure provides a pharmaceutical composition for use in treating homozygous familial hypercholesterolemia (hoFH) in a patient in need thereof, wherein the composition comprises the PCSK9 antibody and a pharmaceutically acceptable excipient.

Other embodiments of the present disclosure will become apparent from a review of the ensuing detailed description.

### BRIEF DESCRIPTION OF THE FIGURES

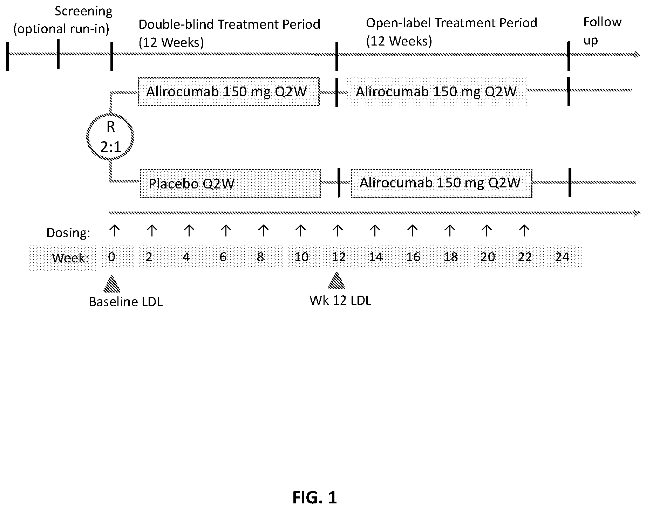

**Figure 1** is a Study Flow Diagram illustrating the clinical trial described in Example 2 herein.

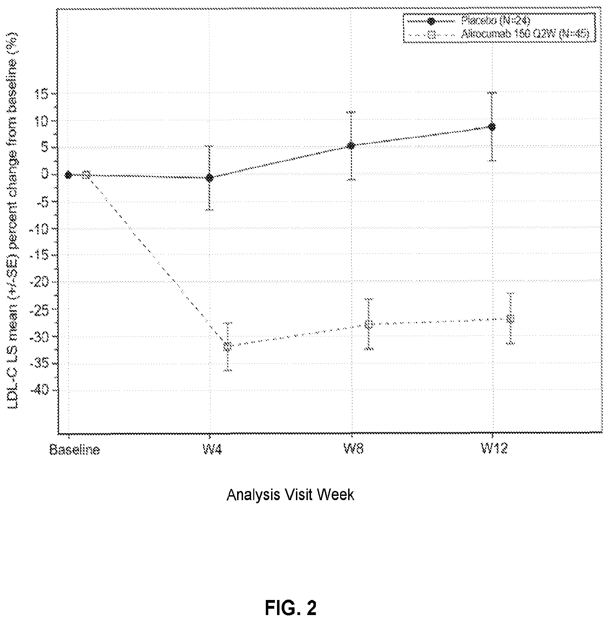

**Figure 2** graphically depicts the LDL-C LS Mean (+/-SE) percent change from baseline for the double-blind period over time. Least-squares (LS) means, standard errors (SE), and p-value taken from MMRM (mixed-effect model with repeated measures) analysis. The model includes the fixed treatment effect, randomization strata as per IVRS, time point, treatment-by-time point interaction, strata-by-time point interaction, as well as the continuous baseline LDL-C value by time-point interaction.

### DETAILED DESCRIPTION

Before the present disclosure is described, it is to be understood that this disclosure is not limited to particular methods and experimental conditions described, as such methods and conditions may vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice of the present disclosure, the preferred methods and materials are now described.

### Statin Inefficacy and Intolerance

In some embodiments, the present disclosure relates generally to compositions that are useful for treating patients who are refractory to treatment with statins, intolerant to statins ("statin intolerant patients," also referred to herein as "a patient who is intolerant to statins"), or who has a history of adverse reactions to statin therapy. As used herein, a patient is regarded as "refractory to statins" if the patient has been subjected to statin therapy without sufficient improvement in the lipid parameters. For example, a patient who is refractory to statins has been on stable statin therapy but still has serum LDL-C levels of at least 70 mg/dL.

As used herein, a patient is regarded as "statin intolerant" or "intolerant to statins" if the patient, has a history of experiencing one or more adverse reactions that began or increased while on a daily statin therapeutic regimen and stopped when statin therapy was discontinued. In certain embodiments, the adverse reactions are musculoskeletal in nature, *e.g*., skeletal muscle pain, aches, weakness or cramping (*e.g*., myalgia, myopathy, rhabdomyolysis, etc.). In certain embodiments, the adverse reactions are skeletal muscle pain or aches that occur or are intensified following exercise or exertion. Statin-related adverse reactions also include hepatic, gastrointestinal and psychiatric symptoms that correlate with statin administration.

According to certain embodiments, a patient is deemed "statin intolerant" or "intolerant to statins" if the patient has a history of skeletal muscle-related symptoms associated with at least two different and separate daily statin therapeutic regimens. According to certain embodiments, a patient is "statin intolerant" or "intolerant to statins" if the patient exhibits one or more statin-related adverse reaction(s) to the lowest approved daily doses of one or more statins. In certain embodiments, a patient is "statin intolerant" or "intolerant to statins" if the patient is unable to tolerate a cumulative weekly statin dose of seven times the lowest approved tablet size. According to other embodiments of the present disclosure, a patient is "statin intolerant" or "intolerant to statins" if the patient is able to tolerate a low dose statin therapy but develops symptoms when the dose is increased (*e.g*., to achieve a targeted LDL-C level).

According to the present disclosure, "a history of skeletal muscle-related symptoms associated with taking at least two different and separate statins" includes skeletal muscle-related pain, aches, weakness and/or cramping, that began or increased during statin therapy and stopped when statin therapy was discontinued. In the context of the present disclosure, exemplary statin therapies associated with statin intolerance may include daily therapeutic statin regimens selected from the group consisting of: 5 mg rosuvastatin daily, 10 mg atorvastatin daily, 10 mg simvastatin daily, 20 mg lovastatin daily, 40 mg pravastatin daily, 40 mg fluvastatin daily, and 2 mg pitavastatin daily.

### Methods for Treating Homozygous Familial Hypercholesterolemia and Reducing Serum LDL-C Levels

According to the invention, the patient who is treatable by the present invention has homozygous Familial Hypercholesterolemia (hoFH) (sometimes referred to herein as "a hypercholesterolemic patient"). Homozygous familial hypercholesterolemia (hoFH) can be characterized by high LDL-cholesterol levels and atherosclerotic cardiovascular disease, despite treatment with lipid-lowering therapies.

In some embodiments, a patient is diagnosed with hoFH based on genotype or clinical criteria. In some embodiments, patients diagnosed with hoFH include all individuals considered to be true homozygotes (same mutation(s) in both alleles of the same gene), compound heterozygotes (different mutations in each allele of the same gene), or double heterozygotes (different mutations in different genes) for mutations in the LDLR, ApoB, PCSK9, or LDLRAP1 genes. In some embodiments, the mutation is characterized as "null" or "non-null" based on the amount of residual LDLR activity. In some embodiments, the patient is diagnosed with hoFH based on a genotype including: (a) homozygous non-null/non-null; (b) compound heterozygous non-null-/non-null; (c) compound heterozygous non-null/null; or (d) homozygous null/null. In some embodiments, a patient having a "null/null" mutation has residual LDLR activity < 2%. In some embodiments, the patient is diagnosed with hoFH based on one or more clinical criteria, including but not limited to: (a) untreated total cholesterol >500 mg/dL (12.93 mmol/L) and triglycerides (TG) <300 mg/dL (3.39 mmol/L), (b) both parents with history of total cholesterol >250 mg/dL (6.46 mmol/L), and(c) cutaneous or tendinous xanthoma before age 10. In some embodiments, a patient having hoFH is selected for treatment with the methods and compositions disclosed herein.

Also disclosed are methods for reducing serum LDL-C levels in a patient having hoFH. The subject may have hoFH and be refractory to treatment with statins, be intolerant to statins, and/or have a history of adverse reactions to statin therapy. Similarly, also disclosed are methods for reducing serum LDL-C levels in a patient having hoFH without inducing skeletal muscle pain, discomfort, weakness, or cramping. As used in this context, "reducing serum LDL-C levels" means causing the patient's serum LDL-C level to decrease by at least 10% (*e.g.*, at least 15%, 20%, 25%, 30%, 35%, 40%, 45%, 50%, 55%, 60%, 65%, 70%, 75% or more). The terms "subject" and "patient" are used interchangeably herein.

### Methods for Eliminating or Reducing Statin Usage

Also disclosed are methods and composition useful, *inter alia,* for eliminating or reducing statin usage in a patient having hoFH. In some embodiments, the patient having hoFH is refractory or intolerant to statins or who experiences adverse events upon treatment with statins. The methods may comprise: (a) selecting a patient who is or was on a daily therapeutic statin regimen and who is refractory to statin therapy, intolerant to statins, or who has a history of adverse reactions to statin therapy; and (b) discontinuing or reducing the patient's daily therapeutic statin regimen; and (c) administering one or more doses of a PCSK9 inhibitor to the patient. The patient's daily therapeutic statin regimen may be completely discontinued at the time of or just prior to commencement of a therapeutic course of treatment comprising administration of one or more doses of a PCSK9 inhibitor to the patient. Alternatively, the patient's daily therapeutic statin regimen may be gradually reduced at the time of or just prior to commencement of a therapeutic course of treatment comprising administration of one or more doses of a PCSK9 inhibitor to the patient. Gradual reduction of a statin regimen, in the context of this aspect of the disclosure, may comprise reducing the quantity of statin administered to a patient, and/or decreasing the frequency of administration of statin to the patient. Gradual reduction of a statin regimen, according to this aspect of the disclosure, may result in complete elimination of statin usage by the patient while the patient is receiving a PCSK9 inhibitor in place of the statin. In this respect, the adverse effects of statins on a patient are reduced or eliminated by reducing or eliminating statin usage by the patient, while still permitting adequate treatment of homozygous familial hypercholesterolemia in the patient by administration of a PCSK9 inhibitor.

### Patient Selection

The present invention provides compositions useful, *inter alia,* for treating patients who have homozygous familial hypercholesterolemia (hoFH), including for example patients who are "refractory to statins", "statin intolerant", or "intolerant to statins", and/or who experience adverse reactions upon treatment with statins (as defined elsewhere herein). The patients who are treatable by the present invention may also exhibit one or more additional selection criteria. For example, a patient may be selected for treatment with the methods of the present disclosure on the basis of having moderate, high, or very high CV risk. Degree of CV risk may be assessed and expressed in terms of a calculated 10-year fatal cardiovascular disease (CVD) risk SCORE value, as defined by The Task Force for the Management of Dislipidaemias of the European Society of Cardiology (ESC) and the European Atherosclerosis Society (EAS), as set forth in the ESC/EAS Guidelines for the Management of Dislipidaemias, European Heart Journal, 2100; 32:1769-1818 (referred to herein as "ESC/EAS 2011").

As used herein, "moderate CV risk" means a calculated 10-year fatal CVD risk SCORE greater than or equal to 1% and less than 5%. As used herein, "high CV risk" means a calculated 10-year fatal CVD risk SCORE greater than or equal to 5%, and/or moderate kidney disease (CKD), and/or type 1 or type 2 diabetes mellitus without target organ damage, and/or heFH. As used herein, "very high CV risk" means a history of documented coronary heart disease (CHD), ischemic stroke, peripheral arterial disease (PAD), transient ischemic attack (TIA), abdominal aortic aneurysm, carotid artery occlusion greater than 50% without symptoms, carotid endarterectomy or carotid artery stent procedure, renal artery stenosis, renal artery stent procedure, and/or type 1 or type 2 diabetes mellitus with target organ damage.

According to certain embodiments, the patient may be selected on the basis of having a history of coronary heart disease (CHD). As used herein, a "history of CHD" (or "documented history of CHD") includes one or more of: (i) acute myocardial infarction (MI); (ii) silent MI; (iii) unstable angina; (iv) coronary revascularization procedure (e.g., percutaneous coronary intervention [PCI] or coronary artery bypass graft surgery [CABG]); and/or (v) clinically significant CHD diagnosed by invasive or non-invasive testing (such as coronary angiography, stress test using treadmill, stress echocardiography or nuclear imaging).

According to certain embodiments, the patient may be selected on the basis of having one or more additional risk factors selected from the group consisting of age (*e.g*., older than 40, 45, 50, 55, 60, 65, 70, 75, or 80 years), race, national origin, gender (male or female), exercise habits (*e.g*., regular exerciser, non-exerciser), other preexisting medical conditions (*e.g*., type-II diabetes, high blood pressure, etc.), and current medication status (*e.g.*, currently taking beta blockers, niacin, ezetimibe, fibrates, omega-3 fatty acids, bile acid resins, etc.).

As used herein, "maximally tolerated statin therapy" or "maximum tolerated dose of statin therapy" are used interchangeably to mean a therapeutic regimen comprising the administration of a daily dose of a statin that is the highest dose of statin that can be administered to a particular patient without causing unacceptable adverse side effects in the patient. Maximally tolerated statin therapy includes, but is not limited to, high intensity statin therapy.

As used herein, "maximally tolerated lipid modifying therapy" or "maximum tolerated LMT" are used interchangeably to mean a therapeutic regimen comprising the administration of a daily, weekly, or monthly dose of a lipid modifying therapy (LMT) that is the highest dose of the LMT that can be administered to a particular patient without causing unacceptable adverse side effects in the patient. Maximally tolerated LMT includes, but is not limited to, high intensity statin therapy, ezetimibe, fibrates, bile acid sequestrants, cholesterol absorption inhibitors, nicotinic acid or derivatives, omega 3 fatty acids, probucol, lomitapide, and mipomersen.

As used herein, the term "premature cardiovascular disease" refers to cardiovascular disease in a patient before the age of 50 years old.

### Therapeutic Efficacy

The present invention results in the reduction in serum levels of one or more lipid component selected from the group consisting of LDL-C, ApoB, non-HDL-C, total cholesterol (TC), triglycerides (TG), Lp(a), and/or remnant cholesterol. For example, according to certain embodiments of the present disclosure, administration of a pharmaceutical composition comprising the PCSK9 inhibitor to a patient with hoFH will result in a mean percent reduction from baseline in serum low density lipoprotein cholesterol (LDL-C) of at least about 25%, 30%, 40%, 45%, 50%, 60%, or greater; a mean percent reduction from baseline in ApoB of at least about 20%, 25%, 30%, 40%, 50%, 60%, or greater; a mean percent reduction from baseline in non-HDL-C of at least about 20%, 25%, 30%, 40%, 50%, 60%, or greater; a mean percent reduction from baseline in total cholesterol of at least about 10%, 15%, 20%, 25%, 30%, 35%, or greater; a mean percent reduction from baseline in triglycerides (*e.g*., fasting triglycerides) of at least about 5%, 10%, 15%, 20%, 25%, 30%, 35% or greater; and/or a mean percent reduction from baseline in Lp(a) of at least about 5%, 10%, 15%, 20%, 25%, 30%, or greater. The percent reductions in the various lipid parameters as set forth above may be achieved at 1, 2, 3, 4, 5, 6, 7, 8, 9, 10, 11, 12, 14, 16, 18, 20, 22, 24, 26, 28, 30, or more weeks after the commencement of a therapeutic regimen comprising the administration of a PCSK9 inhibitor as disclosed herein (*e.g*., 150 mg mAb316P administered once every two weeks, or other similar administration regimens; see, *e.g.,* Example 2 herein).

According to certain specific embodiments, the present invention includes reducing serum LDL-C levels in a patient with hoFH. Accordingf to the invention, the patient having hoFH is refractory to statins, intolerant to statins, or who has a history of adverse reactions to statin therapy. Also disclosed are methods for treating, delaying onset of, and/or reducing the risk of developing atherosclerosis in a patient having homozygous familial hypercholesterolemia (hoFH). These methods may comprise: (a) selecting a patient with moderate, high, or very high cardiovascular risk who is refractory to statins, intolerant to statins, or has a history of adverse reactions to statin therapy; and (b) administering one or more doses of an anti-PCSK9 antibody to the patient at a dosing amount of about 150 mg per dose, and a dosing frequency of about once every two weeks. After about 12 weeks of treatment with the anti-PCSK9 antibody, the patient may exhibit one or more lipid parameter improvements selected from the group consisting of: a reduction in LDL-C level from baseline of about 35%, a reduction in non-HDL-C level from baseline of about 33%, a reduction in Apo B level from baseline of about 30%, a reduction in total cholesterol level from baseline of about 27%, a reduction in (fasting) triglyceride level from baseline of about 11%, and/or a reduction in Lp(a) level from baseline of about 28%. Methods according to this aspect may comprise discontinuing the patient's background statin therapy prior to or concurrent with commencement of treatment with the anti-PCSK9 antibody.

### PCSK9 Inhibitors

The present invention comprises administering to a patient a therapeutic composition comprising the PCSK9 inhibitor. As used herein, the "PCSK9 inhibitor" is a human antibody that specifically binds human proprotein convertase subtilisin/kexin type 9 (hPCSK9) and comprises a heavy chain comprising SEQ ID NO:5 and a light chain comprising SEQ ID NO:9, a heavy chain variable region (HCVR) comprising SEQ ID NO:1 and a light chain variable domain (LCVR) comprising SEQ ID NO:6, wherein the three heavy chain CDRs are set forth in SEQ ID NOs: 2, 3 and 4, and the three light chain CDRs are set forth in SEQ ID NOs: 7, 8 and 10.

The term "human proprotein convertase subtilisin/kexin type 9" or "human PCSK9" or "hPCSK9", as used herein, refers to PCSK9 encoded by the nucleic acid sequence shown in SEQ ID NO:197 and comprising the amino acid sequence of SEQ ID NO:198, or a biologically active fragment thereof.

The term "antibody", as used herein, is intended to refer to immunoglobulin molecules comprising four polypeptide chains, two heavy (H) chains and two light (L) chains inter-connected by disulfide bonds, as well as multimers thereof (*e.g*., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the disclosure, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, *e.g*., commercial sources, DNA libraries (including, *e.g*., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g*., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e.g*. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR, which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

An antigen-binding fragment of an antibody may contain at least one variable domain covalently linked to at least one constant domain. Non-limiting, exemplary configurations of variable and constant domains that may be found within an antigen-binding fragment of an antibody of the present disclosure include: (i) V_{H}-C_{H}L; (ii) V_{H}-C_{H}2; (iii) V_{H}-C_{H}3; (iv) V_{H}-C_{H}l-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g.*, 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present disclosure may comprise a homo-dimer or hetero-dimer (or other multimer) of any of the variable and constant domain configurations listed above in non-covalent association with one another and/or with one or more monomeric V_{H} or V_{L} domain (*e.g.*, by disulfide bond(s)).

As with full antibody molecules, antigen-binding fragments may be monospecific or multispecific (*e.g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present disclosure using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies of the disclosure may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. However, such recombinant human antibodies may be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant disclosure encompasses antibodies having one or more mutations in the hinge, C_{H}2 or C_{H}3 region, which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present disclosure. An isolated antibody also includes an antibody *in situ* within a recombinant cell. Isolated antibodies are antibodies that have been subjected to at least one purification or isolation step. An isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present disclosure, includes antibodies that bind PCSK9 or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9, however, have cross-reactivity to other antigens, such as PCSK9 molecules from other (non-human) species.

The term "surface plasmon resonance", as used herein, refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "K_{D} ", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain embodiments, the anti-PCSK9 antibody used in the present invention is an antibody with pH-dependent binding characteristics. As used herein, the expression "pH-dependent binding" means that the antibody or antigen-binding fragment thereof exhibits "reduced binding to PCSK9 at acidic pH as compared to neutral pH" (for purposes of the present disclosure, both expressions may be used interchangeably). For the example, antibodies "with pH-dependent binding characteristics" include antibodies and antigen-binding fragments thereof that bind PCSK9 with higher affinity at neutral pH than at acidic pH. In certain embodiments, the antibodies bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

As used herein, the expression "acidic pH" means a pH of 6.0 or less (*e.g*., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

One can easily determine whether an antibody binds to the same epitope as, or competes for binding with, a reference anti-PCSK9 antibody by using routine methods known in the art. For example, to determine if a test antibody binds to the same epitope as a reference anti-PCSK9 antibody of the invention, the reference antibody is allowed to bind to a PCSK9 protein or peptide under saturating conditions. Next, the ability of a test antibody to bind to the PCSK9 molecule is assessed. If the test antibody is able to bind to PCSK9 following saturation binding with the reference anti-PCSK9 antibody, it can be concluded that the test antibody binds to a different epitope than the reference anti-PCSK9 antibody. On the other hand, if the test antibody is not able to bind to the PCSK9 molecule following saturation binding with the reference anti-PCSK9 antibody, then the test antibody may bind to the same epitope as the epitope bound by a reference anti-PCSK9 antibody described herein.

To determine if an antibody competes for binding with a reference anti-PCSK9 antibody, the above-described binding methodology is performed in two orientations: In a first orientation, the reference antibody is allowed to bind to a PCSK9 molecule under saturating conditions followed by assessment of binding of the test antibody to the PCSK9 molecule. In a second orientation, the test antibody is allowed to bind to a PCSK9 molecule under saturating conditions followed by assessment of binding of the reference antibody to the PCSK9 molecule. If, in both orientations, only the first (saturating) antibody is capable of binding to the PCSK9 molecule, then it is concluded that the test antibody and the reference antibody compete for binding to PCSK9. As will be appreciated by a person of ordinary skill in the art, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. That is, a 1-, 5-, 10-, 20- or 100-fold excess of one antibody inhibits binding of the other by at least 50% but preferably 75%, 90% or even 99% as measured in a competitive binding assay (see, e.g., Junghans et al., Cancer Res, 1990:50:1495-1502). Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

Non-limiting examples of anti-PCSK9 antibodies that can be used in the context of the present disclosure include, e.g., alirocumab, bococizumab, or antigen-binding portions thereof.

### Preparation of Human Antibodies

Methods for generating human antibodies in transgenic mice are known in the art. Any such known methods can be used in the context of the present disclosure to make human antibodies that specifically bind to human PCSK9.

Using VELOCIMMUNE^{™} technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody of the disclosure, for example wild-type or modified IgG1 or IgG4. While the constant region selected may vary according to specific use, high affinity antigen-binding and target specificity characteristics reside in the variable region.

Sequence identity between two amino acids sequences is determined over the entire length of the reference amino acid sequence, i.e. the amino acid sequence identified with a SEQ ID NO, using the best sequence alignment and/or over the region of the best sequence alignment between the two amino acid sequences, wherein the best sequence alignment can be obtained with art known tools, e.g. Align, using standard settings, preferably EMBOSS::needle, Matrix: Blosum62, Gap Open 10.0, Gap Extend 0.5.

According to the present invention, the antibody comprises the six CDRs (HCDR1, HCDR2, HCDR3, LCDR1, LCDR2 and LCDR3) from the heavy and light chain variable region amino acid sequence pairs (HCVR/LCVR) of SEQ ID NOs:1/6

According to the invention, the anti-PCSK9 antibody has HCDR1/HCDR2/HCDR3/LCDR1/LCDR2/LCDR3 amino acid sequences selected from SEQ ID NOs: 2/3/4/7/8/10 (mAb316P [also referred to as "REGN727," or "alirocumab"]).

### Pharmaceutical Compositions and Methods of Administration

The present disclosure includes the step of administering the PCSK9 inhibitor to a patient, wherein the PCSK9 inhibitor is contained within a pharmaceutical composition. The pharmaceutical compositions of the disclosure are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition of the disclosure, *e.g*., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present disclosure can be delivered subcutaneously or intravenously with a standard needle and syringe. In addition, with respect to subcutaneous delivery, a pen delivery device readily has applications in delivering a pharmaceutical composition of the present disclosure. Such a pen delivery device can be reusable or disposable. Such a pen delivery device can be prefilled. A reusable pen delivery device generally utilizes a replaceable cartridge that contains a pharmaceutical composition. Once all of the pharmaceutical composition within the cartridge has been administered, and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present disclosure. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen delivery devices having applications in subcutaneous delivery of a pharmaceutical composition of the present disclosure include, but are not limited to the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, e.g., Goodson, 1984, in Medical Applications of Controlled Release, supra, vol. 2, pp. 115-138). In some embodiments, the pharmaceutical composition can be contained in a microinfusor. Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc. In some embodiments, the composition is contained in a glass vial.

Exemplary pharmaceutical formulations comprising an anti-PCSK9 antibody that can be used in the context of the methods of the present disclosure are set forth, *e.g*., in US 2013/0189277.

### Dosage

The amount of PCSK9 inhibitor (*e.g*., anti-PCSK9 antibody) administered to a subject according to the present disclosure is, generally, a therapeutically effective amount. As used herein, the phrase "therapeutically effective amount" means a dose of PCSK9 inhibitor that results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of LDL-C, ApoB100, non-HDL-C, total cholesterol, VLDL-C, triglycerides, Lp(a) and remnant cholesterol.

In the case of an anti-PCSK9 antibody, a therapeutically effective amount can be from about 0.05 mg to about 600 mg, *e.g*., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody.

The amount of anti-PCSK9 antibody contained within the individual doses may be expressed in terms of milligrams of antibody per kilogram of patient body weight (i.e., mg/kg). For example, the anti-PCSK9 antibody may be administered to a patient at a dose of about 0.0001 to about 10 mg/kg of patient body weight.

### Combination Therapies

According to certain embodiments of the present disclosure, additional therapeutic agents, besides a statin, may be administered to the patient in combination with a PCSK9 inhibitor. Examples of such additional therapeutic agents include *e.g*., (1) an agent which inhibits cholesterol uptake and or bile acid re-absorption (*e.g*., ezetimibe); (2) an agent which increase lipoprotein catabolism (such as niacin); and/or (3) activators of the LXR transcription factor that plays a role in cholesterol elimination such as 22-hydroxycholesterol. According to certain embodiments, an anti-ANGPTL3 antibody (such as evinacumab) is administered in combination with a PCSK9 inhibitor in the context of the methods of the present disclosure.

### Administration Regimens

According to the present disclosure, one or more doses of a PCSK9 inhibitor (*i.e*., a pharmaceutical composition comprising a PCSK9 inhibitor) may be administered to a subject over a defined time course (*e.g*., in place of a daily therapeutic statin regimen). This comprises sequentially administering to a subject one or more doses of a PCSK9 inhibitor. As used herein, "sequentially administering" means that each dose of PCSK9 inhibitor is administered to the subject at a different point in time, *e.g*., on different days separated by a predetermined interval (*e.g*., hours, days, weeks or months). The present disclosure thus comprises sequentially administering to the patient a single initial dose of a PCSK9 inhibitor, followed by one or more secondary doses of the PCSK9 inhibitor.

The terms "initial dose," "secondary doses," and "tertiary doses," refer to the temporal sequence of administration of the individual doses of a pharmaceutical composition comprising a PCSK9 inhibitor. Thus, the "initial dose" is the dose that is administered at the beginning of the treatment regimen (also referred to as the "baseline dose"); the "secondary doses" are the doses that are administered after the initial dose; and the "tertiary doses" are the doses that are administered after the secondary doses. The initial, secondary, and tertiary doses may all contain the same amount of the PCSK9 inhibitor, but generally may differ from one another in terms of frequency of administration. Two or more (*e.g*., 2, 3, 4, or 5) doses can be administered at the beginning of the treatment regimen as "loading doses" followed by subsequent doses that are administered on a less frequent basis (*e.g*., "maintenance doses").

According to the present invention, each secondary dose is administered 2 weeks after the immediately preceding dose. The phrase "the immediately preceding dose," as used herein, means, in a sequence of multiple administrations, the dose of antigen-binding molecule that is administered to a patient prior to the administration of the very next dose in the sequence with no intervening doses.

The invention may comprise administering to a patient any number of secondary doses of the PCSK9 inhibitor. For example, in certain embodiments, only a single secondary dose is administered to the patient. In other embodiments, two or more (*e.g*., 2, 3, 4, 5, 6, 7, 8, or more) secondary doses are administered to the patient.

According to the present invention, multiple doses of a pharmaceutical composition comprising about 150 mg of the anti-PCSK9 antibody are administered to a patient at a frequency of once every two weeks.

Also disclosed are administration regimens comprising an up-titration option (also referred to herein as "dose modification"). As used herein, an "up-titration option" means that, after receiving a particular number of doses of a PCSK9 inhibitor, if a patient has not achieved a specified reduction in one or more defined therapeutic parameters, the dose of the PCSK9 inhibitor is thereafter increased. For example, in the case of a therapeutic regimen comprising administration of 75 mg doses of an anti-PCSK9 antibody to a patient at a frequency of once every two weeks, if after 8 weeks (i.e., 5 doses administered at Week 0, Week 2 and Week 4, Week 6 and Week 8), the patient has not achieved a serum LDL-C concentration of less than 70 mg/dL, then the dose of anti-PCSK9 antibody is increased to e.g., 150 mg administered once every two weeks thereafter (*e.g*., starting at Week 10 or Week 12, or later).

### EXAMPLES

The following examples are put forth so as to provide those of ordinary skill in the art with a complete disclosure and description of how to make and use the methods and compositions of the disclosure, and are not intended to limit the scope of what the inventors regard as their disclosure. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human PCSK9

Human anti-PCSK9 antibodies were generated as described in US Patent No. 8,062,640. The exemplary PCSK9 inhibitor used in the following Example is the human anti-PCSK9 antibody designated "mAb316P," also known as "REGN727," or "alirocumab." mAb316P has the following amino acid sequence characteristics: a heavy chain comprising SEQ ID NO:5 and a light chain comprising SEQ ID NO:9; a heavy chain variable region (HCVR) comprising SEQ ID NO:1 and a light chain variable domain (LCVR) comprising SEQ ID NO:6; a heavy chain complementarity determining region 1 (HCDR1) comprising SEQ ID NO:2, a HCDR2 comprising SEQ ID NO:3, a HCDR3 comprising SEQ ID NO:4, a light chain complementarity determining region 1 (LCDR1) comprising SEQ ID NO:7, a LCDR2 comprising SEQ ID NO:8 and a LCDR3 comprising SEQ ID NO:10.

### Example 2: A Randomized, Double-Blind, Placebo-controlled, Parallel-group Study to Evaluate the Efficacy and Safety of an Anti-PCSK9 Antibody ("alirocumab") in Patients with Homozygous Familial Hypercholesterolemia

The objective of the instant study was to evaluate the efficacy, safety and tolerability of an anti-PCSK9 antibody ("alirocumab") in patients with hoFH (excluding those patients with null/null mutations in both LDLR alleles). More specifically, an objective of the instant study was to demonstrate the reduction of LDL-C with alirocumab 150 mg subcutaneous (SC) every 2 weeks (Q2W) in comparison to placebo after 12 weeks of treatment. Secondary objectives of the study included: evaluating the effect of alirocumab 150 mg Q2W on other lipid parameters (i.e., apolipoprotein [Apo] A-1 and B, non-high-density lipoprotein cholesterol [non HDL C], total cholesterol [TC], proportion of patients with 15%, 30%, and 50% LDL-C reductions, Lp(a), HDL-C, triglycerides [TG]) in patients with hoFH), evaluating the safety and tolerability of alirocumab 150 mg SC Q2W in patients with hoFH, assessing the pharmacokinetics (PK) of alirocumab 150 mg SC Q2W in patients with hoFH, and assessing the potential development of anti-drug (alirocumab) antibodies.

Other objectives of the instant study included: collecting genotype information for all patients to characterize hoFH mutation status in order to explore potential differences in efficacy and safety, assessing the effect of alirocumab on eligibility for apheresis (using German and US apheresis criteria), and assessing the effect of alirocumab on quality of life using the EQ-5D QOL questionnaire.

Patients with hoFH have persistently elevated LDL-C, which is a contributing factor to a number of health concerns, principally accelerated atherosclerosis resulting in premature CV disease. Despite treatment with LMTs such as pharmacological agents including statins, and mechanical removal of lipids by LDL apheresis, many patients with hoFH remain far from their LDL-C treatment goal. In a cohort study, the age of first CVD event was in the third decade of life in hoFH (Raal, et al. 2011 Circulation 124(20):2202-2207). While this is later than the early teen years as seen before implementation of medical regimens such as statins, the need for more intensive treatments remain in order to delay the onset of CVD and occurrence of events.

In order to evaluate the efficacy and safety of alirocumab in patients with hoFH, the instant study was carried out. The study population included individuals ≥18 years of age. Diagnosis of hoFH was based on either genotyping data or clinical criteria. The genetic definition included all individuals considered to be true homozygotes, compound heterozygotes, or double heterozygotes for mutations in the LDLR, ApoB, PCSK9, or LDLRAP1 genes. However, individuals with history null/null LDLR mutations were excluded.

Percent change in LDL-C from baseline was the primary endpoint. Low-density lipoprotein cholesterol is an accepted surrogate endpoint for CV risk and has repeatedly been used as the primary endpoint for approval of multiple other hoFH treatments. The instant study was designed as a placebo controlled trial, with the addition of alirocumab on top of patients' existing treatment regimens of maximally tolerated LMT, including lipid apheresis. An optional run-in period was utilized for those patients that have not yet achieved a stable background treatment regimen that would be required to be maintained throughout the double-blind treatment period. An add-on design was appropriate, because removal of any therapies from the patient's existing treatment regimen would lead to an increase in LDL-C and possibly contribute to the serious CV sequelae seen in this disease. Treatment duration of 12 weeks for the primary endpoint allowed alirocumab to achieve steady state and exert its full effect on LDL C. An additional 12-week open-label treatment period in which all patients were administered alirocumab allowed further assessment of safety in this population.

### Rationale for Dose Selection

Alirocumab 75 mg and 150 mg SC Q2W are the approved doses and is currently authorized in 40 countries worldwide (including the US, European Union, Canada, Norway, Iceland, Brazil, and Japan). Because patients with hoFH are proven to be a hard-to-treat population compared to non-FH and HeFH patients and will have a very high baseline LDL-C far from their target level, the dose of alirocumab proposed for the instant study is the highest approved dose, 150 mg SC Q2W.

In addition to being efficacious, alirocumab has a favorable safety profile. Overall, the most commonly occurring treatment-emergent adverse events (TEAEs), reported in a higher proportion of patients in the alirocumab group compared to placebo (i.e., incidence ≥2.0% in the alirocumab group) were: injection site reaction (7.2% vs 5.1%), nasopharyngitis (11.3% vs 11.1%), influenza (5.7% vs 4.6%), myalgia (4.3% vs 3.4%), urinary tract infection (4.8% vs 4.6%), diarrhea (4.7% vs 4.4%) and bronchitis (4.3% vs 3.8%) (Praluent Product Insert). No differences in the safety profile have been observed between the two approved doses of 75 mg and 150 mg.

It was hypothesized that treatment with alirocumab 150 mg Q2W in patients with hoFH already receiving maximally tolerated LMT or LDL apheresis would be well-tolerated and have an acceptable safety profile while providing maximal LDL-C lowering effect.

### Benefit / Risk Assessment

Patients with hoFH have extremely high LDL-C levels, are far from their target LDL-C, and require significant reductions in LDL-C. Despite the approval of newer treatments including lomitapide and mipomersen, the need for more intensive therapies remain. PCSK9 inhibitors are a new addition to the armamentarium of LMT that has proven to profoundly decrease LDL-C. The body of evidence from the statin literature shows that the relationship between LDL-C reduction and CV event reduction is approximately linear, and within the context of the instant study in the hoFH patient population, even a modest reduction in LDL-C would translate into significant benefit for these patients.

It was expected that treatment with alirocumab would be well tolerated and have an acceptable safety profile. The accumulated safety information showed that the most commonly occurring TEAEs with alirocumab were nasopharyngitis, injection site reactions, influenza, myalgia, musculoskeletal pain, and contusion. Moreover, the rates of these adverse events (AEs) were relatively low, ranging from 11.3% to 2.1% for the alirocumab treatment group (vs 11.1% - 1.6% in the placebo group) (Praluent Product Insert).

Taken together, these data indicated that the benefit/risk assessment of treatment with alirocumab in the hoFH populations could be favorable.

### Demographic and Baseline Characteristics

Baseline characteristics included standard demography (age, race, weight, height, etc), disease characteristics including lipid levels, mutation status, medical history, medication history and apheresis schedule (if applicable) for each patient.

### Primary and Secondary Endpoints

The primary efficacy endpoint was the percent change in LDL-C from baseline to week 12 in the ITT population for alirocumab 150 mg Q2W as compared with placebo in patients with hoFH. The percent change in LDL-C from baseline to week 12 was defined as: [100 x (LDL-C value at week 12 -LDL-C value at baseline)]/LDL-C value at baseline. For LDL-C analysis, both calculated and measured LDL-C values were taken into account. In case both calculated and measured LDL-C values were available for the same sampling time point, the measured LDL-C was considered. The baseline LDL-C value was the last LDL C value obtained before the first dose of double blind-study drug. For randomized but not-treated patients, baseline was defined as the last value before randomization. The LDL C at week 12 was the LDL-C value obtained within the week 12 analysis window, regardless of adherence to treatment (ITT estimand).

All calculated and measured LDL-C values (scheduled or unscheduled, fasting or not fasting) could be used for the primary efficacy endpoint, if appropriate, according to the above definition. The analysis window used to allocate a time point to a measurement was defined in the statistical analysis plan (SAP).

Key secondary efficacy endpoints included: the percent change in Apo B from baseline to week 12 (ITT estimand), the percent change in non-HDL-C from baseline to week 12 (ITT estimand), the percent change in total cholesterol from baseline to week 12 (ITT estimand), proportion of patients with ≥15% reduction in LDL-C at week 12 (ITT estimand), proportion of patients with ≥30% reduction in LDL-C at week 12 (ITT estimand), the percent change in Lp(a) from baseline to week 12 (ITT estimand), proportion of patients with >_50% reduction in LDL-C at week 12 (ITT estimand), the percent change in HDL-C from baseline to week 12 (ITT estimand), the percent change in fasting TG from baseline to week 12 (ITT estimand), and the percent change in Apo A-1 from baseline to week 12 (ITT estimand). The same definition and rules applied to these key secondary efficacy endpoints as were applied to the primary efficacy endpoint.

Other secondary efficacy endpoints included the percent change in LDL-C from baseline to week 12 in the modified (m)ITT population (all randomized population who took at least 1 dose or part of a dose of double-blind investigational study drug and has an evaluable primary endpoint), using all LDL-C values within the week 12 analysis window and during the efficacy treatment period (on-treatment estimand), the percent change in Apo B, non-HDL-C, TC, Lp(a), HDL-C, fasting TG and Apo A-1 from baseline to week 12 (on-treatment estimand), proportion of patients with ≥15% reduction, ≥30% reduction, and >_50% reduction in LDL-C at week 12 (on treatment estimand), and the absolute change in the ratio of Apo B/Apo A-1 from baseline to week 12 (ITT estimand). The efficacy treatment period was defined as the time from the first double-blind study drug injection up to 21 days after the last double-blind study drug injection, or the first open-label alirocumab injection (if applicable), whichever came first.

Safety endpoints constituted safety parameters (AEs, laboratory data, vital signs, and electrocardiogram [ECG]) assessed throughout the study. Other endpoints included exploratory relationships between hoFH genotype status and lipid parameters, the change in the proportion of patients who meet US apheresis eligibility criteria from baseline to week 12 (Goldberg, et al. 2011 J Clin Lipidol 5(3 Supp):S1-S8), the change in the proportion of patients who meet German apheresis eligibility criteria from baseline to week 12 (Schettler, et al. 2012 Clin Res Cardiol Suppl 7:15-19), and response of each EQ-5D item, index score, and change of index score from baseline through week 12.

### Pharmacokinetic Variables

The pharmacokinetic (PK) variable was alirocumab serum concentration collected at specified sampling time.

### Anti-Drug Antibody Variables

Anti-drug (alirocumab) antibody status were assessed: total patients negative in the ADA assay at all time points, pre-existing immunoreactivity (defined as either an anti-drug antibody (ADA) positive response in the assay at baseline with all post-dose ADA results negative OR a positive response at baseline with all post-treatment ADA responses less than 4-fold baseline titer levels), and/or treatment emergent (defined as either any post-dose positive ADA response when baseline results were negative OR any post-dose positive ADA response that was at least 4-fold over the baseline level when baseline was positive in the ADA assay).

Samples positive in the ADA assay were assessed for titer. The titer categories included low (titer <1,000), moderate (1,000≤ titer ≤10,000), and high (titer >10,000).

Samples positive in the ADA assay were assessed for neutralizing activity.

### Study Design

The instant study was a randomized, double-blind, placebo-controlled, parallel-group study to evaluate the efficacy and safety of alirocumab in patients with hoFH.

Approximately 74 patients were randomized in a 2:1 ratio to receive either alirocumab 150 mg SC Q2W or matching placebo. Randomization was stratified by apheresis treatment status (Yes/No).

The study consisted of up to 4 periods: an optional 4-week run-in period (for patients whose background medical LMT regimen or apheresis schedule and/or apheresis settings were stable prior to screening), a 2-week screening period, a 12-week double-blind treatment period, and a mandatory 12-week open-label treatment period according to the following study flow chart:

| | **Optional Run-in** | | **Screening** | | | **Open-Label Treatment** | | |
|---|---|---|---|---|---|---|---|---|
| | | | Baseline | End of Double-Blind | End of Open-Label | | End of Study | |
| (Day -42) | | (Day -14) | (Day 1) | (Day 85) | (Day 169) | | (Day 225) | |

Patients not continuing on to another lipid lowering study also underwent an 8-week follow-up period.

Optional run-ins included undergoing apheresis therapy and lipid-modifying therapy.

Patients who were undergoing apheresis therapy had to be on a stable weekly or every other week schedule. Patients whose schedule or apheresis settings had not been stable for at least 8 weeks before the screening visit entered a 4-week run-in period before the screening period. After the 4-week run-in period, patients whose lipid-apheresis schedule/settings remained stable (and were stable for at least 8 weeks in total) were eligible to enter the 2-week screening period. Additionally, all patients on LDL apheresis had to be diagnosed based on genotype and, if genotype information had not been determined previously, they could enter the run-in to allow time, if needed, to determine their mutation status.

Patients who were on background lipid-modifying therapy (LMT) that had not been stable for at least 4 weeks before the screening visit entered a 4-week run-in period to stabilize their LMT before entering the screening period. Patients who had not been on a stable dose of mipomersen for 6 months prior to screening or on a maximum tolerated dose of lomitapide for 12 weeks prior to screening were excluded.

### Screening

Once on a stable background regimen as defined above, patients entered a 2-week screening period. Initial eligibility was determined during this screening period by standard screening procedures. A DNA sample was collected for hoFH mutation status.

Patients were to be on a stable low fat or heart-healthy diet throughout the duration of the study, starting at screening through the end of the double-blind treatment period and through the open label treatment period. Patients' exercise regimen was to remain stable throughout the duration of the study, from screening, through the end of the double-blind treatment period and through the open-label treatment period.

The patient or caregiver was trained to self-inject/inject using a dose of placebo during the screening period or at the first visit of the double-blind treatment period.

### Double-blind Treatment

Patients who met all inclusion criteria AND who met none of the exclusion criteria were randomized in a 2:1 ratio to receive: alirocumab 150 mg SC Q2W OR matching placebo SC Q2W. What was received is also referred to herein as "study drug" and "investigational medical product".

Study drug administration during the double-blind treatment period started on the day of randomization and was administered immediately after completion of the LDL apheresis procedure (if applicable). For those patients not undergoing LDL apheresis, administration of study drug was made after all samples for clinical laboratory evaluation were obtained. The last injection during the double-blind treatment period was on day 71/week 10.

For all patients undergoing LDL apheresis, all samples for clinical laboratory evaluation were obtained immediately prior to the LDL apheresis procedure and prior to administration of study drug. Given the impact of LDL apheresis on lipid parameters, it was important to match the time of the baseline activities with the timing of the week 12 activities. This meant that timing between the baseline sample collection relative to the most recently completed LDL apheresis procedure should match the timing of the week 12 sample collection relative to the most recently completed LDL apheresis procedure.

For all patients who were not undergoing apheresis, all samples for clinical laboratory evaluation were obtained prior to administration of investigational medical product. The efficacy of alirocumab was assessed by clinical laboratory evaluation of lipid levels at pre-specified time points throughout the study.

Patients who were receiving LMT or who were undergoing apheresis maintained stable LMT and a stable apheresis schedule (as applicable) throughout the duration of the study, from screening through the end of the open-label treatment period/end-of-study visit (week 24).

Patients who prematurely discontinued study drug during the double-blind treatment period were encouraged to remain in the study and undergo all double-blind study visits and procedures with the exception of dosing with study drug. At the time of study drug discontinuation, the patient was to have, as soon as possible, an unscheduled visit with assessments normally planned at the end of the double-blind treatment visit (this was to take place within 5 days of discontinuation of study drug, if possible).

### Open-label Treatment

To provide further safety data in this rare patient population, all patients participated in an open-label treatment period. Regardless of treatment assignment in the double-blind treatment period, patients received open-label study drug (alirocumab 150 mg SC Q2W) starting at week 12 (day 85) and continuing through week 24 (end of open-label treatment period, last injection of study drug on day 155/week 22). Patients who were receiving LMT or who were undergoing LDL apheresis continued a stable dose and regimen and a stable LDL apheresis schedule and settings (as applicable) throughout the duration of the open-label treatment period.

Upon completion of the open-label treatment period, patients either participated in an additional lipid-lowering clinical trial or directly underwent an 8-week follow-up period. A follow-up phone call was made at week 32 to collect AE and concomitant medication information.

The end of study for the instant study was defined as the last visit of the last patient.

### Patient Selection

69 patients with a 2:1 randomization to alirocumab and placebo were included in the study.

Patients who did not meet eligibility criteria during the initial screening were able to rescreen only once. Patients who were rescreened after the screening window ended had to re-consent for study participation and repeat all screening procedures. Patients who did not meet all eligibility criteria during the initial screening and were still within the screening window were able to retest those assessments that did not meet eligibility criteria once.

### Study Population

The study population will consist of male and female patients, ≥18 years of age, diagnosed with hoFH, except for patients known to have null/null mutations in both LDLR alleles.

### Inclusion Criteria

In order to be eligible for the instant study, the patient had to meet the following criteria:
i) males and females ≥18 years of age at the time of the screening visit,
ii) diagnosis of hoFH by at least 1 of the following genotype or clinical criteria (all patients on LDL apheresis to be diagnosed based on genotype): a) documented homozygous or compound heterozygous mutations in both LDLR alleles (note: patients with known null mutations in both LDLR alleles were excluded (see Exclusion Criteria, below), b) presence of homozygous or compound heterozygous mutations in Apo B, PCSK9 or LDLRAP1, c) presence of double heterozygous mutations, i.e., mutations on different genes in the LDLR, Apo B or PCSK9 alleles, d) untreated TC >500 mg/dL (12.93 mmol/L) and TG <300 mg/dL (3.39 mmol/L), and e) both parents with history of TC >250 mg/dL (6.46 mmol/L) OR cutaneous or tendinous xanthoma before age 10,
iii) receiving a stable dose of a statin at the screening visit (note: patients who were not able to tolerate a statin or if statins were found to be ineffective could be included in the study, but the reason was to be documented in the case report form (CRF)),
iv) if undergoing LDL apheresis, must have initiated LDL apheresis at least 3 months prior to screening and must have been on a stable weekly (every 7 days) or every other week (every 14 days) schedule or stable settings for at least 8 weeks,
v) willing to maintain a stable low fat or heart-healthy diet for the duration of the study,
vi) willing and able to comply with clinic visits and study related procedures, and
vii) provided signed informed consent.

A summary of the instant study's patient characteristics at baseline is provided in the following table:

**Table 1: Demographics and Patient Characteristics at Baseline - Randomized Population**

| | | Placebo (N=24) | Alirocumab 150 Q2W (N=45) | All (N=69) | P-Value vs. Placebo |
|---|---|---|---|---|---|
| Age (years) | | | | | |
| | Number | 24 | 45 | 69 | 0.5081 |
| | Mean (SD) | 45.4 (1580) | 42.3 (14.13) | 43.4 (14.69) | |
| | Median | 43.0 | 42.0 | 42.0 | |
| | Min : Max | 23 : 81 | 19 : 69 | 19 : 81 | |
| | | | | | |

| Age group (years) [n (%)] | | | | | |
|---|---|---|---|---|---|
| | Number | 24 | 45 | 69 | 0.6210 |
| | <45 | 13 (54.2%) | 27 (60.0%) | 40 (580%) | |
| | >=45 to <65 | 7 (29.2%) | 14 (31.1%) | 21 (30.4%) | |
| | >=65 to <75 | 3 (12.5%) | 4 (8.9%) | 7 (10.1%) | |
| | >=75 | 1 (4.2%) | 0 | 1 (1.4%) | |
| | | | | | |

| Age group (years) [n (%)] | | | | | |
|---|---|---|---|---|---|
| | Number | 24 | 45 | 69 | 0.4354 |
| | <65 | 20 (83.3%) | 41 (91.1%) | 61 (88.4%) | |
| | >=65 | 4 (16.7%) | 4 (8.9%) | 8 (11.6%) | |
| | | | | | |

| Sex [n (%)] | | | | | |
|---|---|---|---|---|---|
| | Number | 24 | 45 | 69 | 0.6183 |
| | Male | 13 (54.2%) | 21 (46.7%) | 34 (49.3%) | |
| | Female | 11 (45.8%) | 24 (53.3%) | 35 (50.7%) | |

| | | Placebo (N=24) | Alirocumab 150 Q2W (N=45) | All (N=69) | P-Value vs. Placebo |
|---|---|---|---|---|---|
| Race [n (%)] | | | | | |
| | Number | 24 | 45 | 69 | 0.4188 |
| | White | 18 (750%) | 36 (800%) | 54 (78.3%) | |
| | Black or African American | 0 | 2 (4.4%) | 2 (2.9%) | |
| | Asian | 5 (20.8%) | 7 (15.6%) | 12 (17.4%) | |
| | American Indian or Alaska Native | 0 | 0 | 0 | |
| | Native Hawaiian or Other Pacific Islander | 0 | 0 | 0 | |
| | Other | 1 (4.2%) | 0 | 1 (1.4%) | |
| | | | | | |

| Ethnicity [n (%)] | | | | | |
|---|---|---|---|---|---|
| | Number | 24 | 45 | 69 | 0.5396 |
| | Hispanic or Latino | 0 | 2 (4.4%) | 2 (2.9%) | |
| | Not Hispanic or Latino | 24 (100%) | 43 (95.6%) | 67 (97.1%) | |
| | | | | | |

| Weight (kg) | | | | | |
|---|---|---|---|---|---|
| | Number | 24 | 45 | 69 | 0.7913 |
| | Mean (SD) | 70.1 (17.75) | 71.6 (20.23) | 71.1 (1928) | |
| | Median | 69.8 | 71.0 | 70.0 | |
| | Min : Max | 46 : 114 | 42 : 157 | 42 : 157 | |
| | | | | | |

| Weight group (kg) [n (%)] | | | | | |
|---|---|---|---|---|---|
| | Number | 24 | 45 | 69 | 0.8480 |
| | <50 | 2 (8.3%) | 6 (13.3%) | 8 (11.6%) | |
| | >=50 to <70 | 10 (41.7%) | 14 (31.1%) | 24 (34.8%) | |
| | >=70 to <100 | 11 (45.8%) | 22 (48.9%) | 33 (47.8%) | |
| | >=100 | 1 (4.2%) | 3 (6.7%) | 4 (5.8%) | |
| | | | | | |

| BMI (kg/m²) | | | | | |
|---|---|---|---|---|---|
| | Number | 24 | 45 | 69 | 0.9899 |
| | Mean (SD) | 25.1 (5.12) | 25.1 (5.37) | 25.1 (5.24) | |
| | Median | 24.8 | 25.1 | 24.8 | |
| | Min : Max | 18 : 40 | 18 : 47 | 18 : 47 | |
| | | | | | |

| BMI group (kg/m²) [n (%)] | | | | | |
|---|---|---|---|---|---|
| | Number | 24 | 45 | 69 | 1.0000 |
| | <30 | 21 (87.5%) | 40 (88.9%) | 61 (88.4%) | |
| | >=30 | 3 (12.5%) | 5 (11.1%) | 8 (11.6%) | |
| | | | | | |
| Note: p-values comparing baseline data between treatment groups are provided for descriptive purpose, as a screening tool, using Fisher exact test for qualitative data and the asymptotic one-way ANOVA test for Wilcoxon scores (Kruskal-Wallis test) for continuous data. | | | | | |

### Exclusion Criteria

A patient meeting any of the following criteria was excluded from the instant study:
i) documented evidence of a null mutation in both LDLR alleles,
ii) use of a PCSK9 inhibitor within 10 weeks from screening visit,
iii) background medical LMT that has not been stable for at least 4 weeks (6 weeks for fibrates, 24 weeks for mipomersen, 12 weeks for maximum tolerated dose of lomitapide) before the screening visit; patients had the option to enter the optional run-in period; once the patient was stable on his/her background medical LMT for the appropriate amount of time, the patient could enter the screening period,
iv) LDL apheresis schedule/apheresis settings that had not been stable for at least 8 weeks before the screening visit or an apheresis schedule/settings that was not anticipated to be stable over the next 24 weeks; patients had the option to enter the optional run-in period; once the patient was stable on his/her background lipid apheresis schedule/settings for the appropriate amount of time, the patient could enter the screening period,
v) use of nutraceuticals or over-the-counter (OTC) therapies known to affect lipids, at a dose/amount that had not been stable for at least 4 weeks prior to the screening visit or between the screening and randomization visits; patients had the option to enter the optional run-in period; once the patient was stable on his/her nutraceuticals or OTC therapies for the appropriate amount of time, the patient could enter the screening period,
vi) presence of any clinically significant uncontrolled endocrine disease known to influence serum lipids or lipoproteins; this could include newly diagnosed (within 3 months prior to randomization visit [week 0/day 1]) diabetes mellitus, or signs and symptoms of hypothyroidism; as a note, patients on thyroid replacement therapy could be included, if the dosage of replacement therapy had been stable for at least 12 weeks prior to screening and the thyroid stimulating hormone (TSH) level was within the normal range at the screening visit,
vii) unstable weight (variation >5 kg) within 2 months prior to the screening visit (week -2),
viii) initiation of a new diet or major change to a previous diet within 4 weeks prior to screening,
ix) chronic use of systemic corticosteroids, unless on a stable regimen of 10 mg daily prednisone equivalent or less for at least 6 weeks prior to randomization; as a note, topical, intra-articular, nasal, inhaled and ophthalmic steroid therapies were not considered as 'systemic' and were allowed,
x) use of estrogen or testosterone therapy unless the regimen had been stable in the past 6 weeks prior to the screening visit and there were no plans to change the regimen during the study,
xi) systolic blood pressure >160 mmHg or diastolic blood pressure >100 mmHg at the screening visit (1 repeat measurement was allowed),
xii) LDL-C level <70 mg/dL (1.81 mmol/L) at the screening visit,
xiii) history of a MI, unstable angina leading to hospitalization, coronary artery bypass graft surgery, percutaneous coronary intervention, uncontrolled cardiac arrhythmia, carotid surgery or stenting, stroke, transient ischemic attack, valve replacement surgery, carotid revascularization, endovascular procedure or surgical intervention for peripheral vascular disease within 3 months prior to the screening visit,
xiv) history of New York Heart Association (NYHA) class IV heart failure within 12 months before screening,
xv) history of cancer within the past 2 years, except for adequately treated basal cell skin cancer, squamous cell skin cancer, or *in situ* cervical cancer,
xvi) use of any active investigational drugs within 1 month or 5 half-lives prior to the screening visit, whichever is longer,
xvii) conditions/situations such as any clinically significant abnormality identified at the time of screening that, in the judgment of the investigator or any sub-investigator, would preclude safe completion of the study or constrain endpoints assessment; *e.g*., major systemic diseases, patients with short life expectancy, OR considered by the investigator or any sub-investigator as inappropriate for the instant study for any reason, e.g., deemed unable to meet specific protocol requirements, such as scheduled visits, deemed unable to tolerate injections as per the patient or the investigator, investigator or any sub-investigator, pharmacist, study coordinator, other study staff or relative thereof directly involved in the conduct of the protocol, etc., or presence of any other conditions (*e.g*., geographic or social), either actual or anticipated, that the investigator feels would restrict or limit the patient's participation for the duration of the study,
xviii) laboratory findings during screening period (not including randomization labs): hepatitis B surface antigen and/or Hepatitis C antibody (associated with a positive HCV RNA polymerase chain reaction) at the screening visit, positive serum beta-human chorionic gonadotropin (hCG) or urine pregnancy test in women of childbearing potential, estimated glomerular filtration rate (eGFR) <30 mL/min/1.73 m² (calculated by central lab), alanine aminotransferase (ALT) or aspartate aminotransferase (AST) >3 x upper limit of normal (ULN) (1 repeat lab was allowed), or unexplained serum creatine phosphokinase CPK >5 x ULN (1 repeat lab was allowed),
xix) known hypersensitivity to monoclonal antibody therapeutics,
xx) member of the clinical site study team and/or his/her immediate family,
xxi) pregnant or breastfeeding women, and
xxii) women of childbearing potential* who were unwilling to practice highly effective contraception prior to the initial dose/start of the first treatment and for the duration of the study; highly effective contraceptive measures included stable use of combined (estrogen and progestogen containing) hormonal contraception (oral, intravaginal, transdermal) or progestogen-only hormonal contraception (oral, injectable, implantable) associated with inhibition of ovulation initiated 2 or more menstrual cycles prior to screening; intrauterine device (IUD); intrauterine hormone releasing system (IUS); bilateral tubal ligation; vasectomized partner; and or sexual abstinence**. *Postmenopausal women had to be amenorrheic for at least 12 months in order not to be considered of childbearing potential. Pregnancy testing and contraception were not required for women with documented hysterectomy or oophorectomy. **Sexual abstinence was considered a highly effective method only if defined as refraining from heterosexual intercourse during the entire period of risk associated with the study treatments.

### Premature Withdrawal from the Study

Patients had the right to withdraw from the study at any time, for any reason, and without repercussion. The investigator and/or sponsor had the right to withdraw a patient from the study if it was no longer in the interest of the patient to continue in the study, or if the patient's continuation in the study placed the scientific outcome of the study at risk (*e.g*., if a patient did not or could not follow study procedures). An excessive rate of withdrawals would render the study uninterpretable; therefore, unnecessary withdrawal of patients was to be avoided.

The investigator was to make the best effort to contact any patient (*e.g*., contacting patient's family or private physician, reviewing available registries or health care database) who failed to return to the site and to determine health status, including vital status at a minimum. Attempts to contact such patients were to be documented in the patient's records (*e.g*., times and dates of attempted telephone contact, receipt for sending a registered letter).

Patients prematurely discontinued from the study were not replaced.

### Study Treatments

The investigational study drug injections were provided in prefilled pens and were administered SC into the abdomen, thigh, or outer area of the upper arm. The patient or caregiver used placebo for injection training at the clinical site. After study eligibility was confirmed, the patient or caregiver was trained to self-inject/inject using placebo.

### Investigational and Reference Treatments

Double-blind-Treatment: study drug administration during the double-blind treatment period started on the day of randomization and was administered immediately after completion of the LDL apheresis procedure (if applicable). For those patients not undergoing LDL apheresis, administration of the investigational study drug was made after all samples for clinical laboratory evaluation had been obtained. The last injection of double-blind study drug occurred on day 71/week 10. If a dose was missed, the patient was instructed to administer the injection within 7 days from the missed dose. If the missed dose was not administered within 7 days, the patient was instructed to skip the dose and resume the original schedule.

Patients were randomized in a 2:1 ratio to receive: alirocumab 150 mg SC Q2W OR matching placebo SC Q2W. Sterile alirocumab drug product was supplied at a concentration of 150 mg/mL in a prefilled pen. Placebo was also supplied in a prefilled pen.

Open-label Treatment: to provide further safety data in this rare patient population, all patients received open-label investigational study drug (alirocumab 150 mg SC Q2W), starting at week 12 and continuing through week 24 (end-of open-label treatment period/EOS visit, last injection at week 22), regardless of treatment assignment in the double-blind treatment period. Patients who were receiving LMT or who were undergoing apheresis were to continue a stable dose and regimen and a stable apheresis schedule and settings (as applicable) throughout the duration of the open-label treatment period. Sterile alirocumab drug product was supplied at a concentration of 150 mg/mL in a prefilled pen.

### Run-in (optional) and Background Treatment(s)

Apheresis therapy: patients who were undergoing apheresis therapy without a stable weekly or every other week schedule or stable settings for at least 8 weeks before the screening visit entered a 4-week optional run-in period before the screening period. After the 4-week run-in period, patients whose lipid apheresis schedule/settings remain stable were eligible to enter the 2-week screening period. Additionally, all patients on LDL apheresis had to be diagnosed based on genotype and, if genotype information had not been determined previously, they could enter the run-in to allow time, if needed, to determine their mutation status.

Lipid modifying therapy: patients who were on background LMT that had not been stable for at least 4 weeks before the screening visit entered a 4-week run-in period to stabilize their LMT. Patients who had not been on a stable dose of mipomersen within 6 months prior to screening or a maximum tolerated dose of lomitapide for 12 weeks prior to screening were excluded.

### Dose Modification and Study Treatment Discontinuation

Dose modification for an individual patient was not allowed.

Study drug was to be continued whenever possible. In the event the investigational study drug dosing was stopped, it was to be determined if the stop could be made temporarily; permanent discontinuation was to be a last resort. In any case, the patient should remain in the study as long as possible.

Patients who permanently discontinued study drug during the double-blind treatment period were to remain in the study and undergo all double-blind study visits and procedures with the exception of dosing with study drug. At the time of study drug discontinuation, the patient was to have, as soon as possible, an unscheduled visit with assessments normally planned at end of double-blind treatment visit (within 5 days of discontinuation of study drug, if possible). Then, patients were to resume the original study schedule until the end of the double-blind treatment period and all efforts were to be made to perform the week 12 assessments at week 12. The original study schedule continued until the end of the study visit (*i.e*., follow up phone call visit).

Patients who permanently discontinued study drug during the open-label period were to have, as soon as possible, an unscheduled visit with assessments normally planned at the end of the open label treatment period (within 5 days of discontinuation of the study drug, if possible). Upon completion of this visit, the original study schedule resumed until end of study (*i.e*., follow-up phone visit).

Patients permanently discontinued study drug for the following reasons: for female patients, individuals that had become pregnant, were actively trying to become pregnant, or discontinued use of protocol-defined methods of effective birth control, acute injection reaction of clinical concern, at patient request, if, in the investigator's opinion, continuation of the investigational study drug dosing would be detrimental to the patient's well-being, intercurrent condition that required discontinuation of the investigational study drug, at the specific request of the sponsor, and/or patient received double-blind treatment before randomization.

Temporary discontinuation of the investigational study drug was considered by the investigator because of suspected AEs, including allergic events related to the dose of the investigational study drug. Reinitiating the investigational study drug dosing was done under close and appropriate clinical and/or laboratory monitoring. Temporary discontinuation of the investigational study drug is defined as 1 or more scheduled injections that were not administered to the patient as decided by the investigator.

### Management of Acute Reactions

Acute systemic reactions following injection of the investigational study drug (subcutaneous [SC]) were to be treated using clinical judgment to determine the appropriate response according to typical clinical practice.

### Method of Treatment Assignment

The randomized list of treatment kit numbers was generated centrally. An interactive voice response system (IVRS) and/or interactive web response system (IWRS) was used in the instant study. The investigational study drug was packaged in accordance with this list.

Patients were randomly assigned to receive alirocumab 150 mg or matching placebo in a 2:1 ratio, stratified by LDL apheresis treatment status (on vs off treatment).

The treatment kit numbers were allocated using the centralized treatment allocation system at the randomization visit, at weeks specified in Table 1, below, as re-supply visits, and at unscheduled visits, if needed.

### Blinding

Study patients, the investigators, and study site personnel remained blinded to all randomization assignments throughout the instant study. The study director, medical monitor, study monitor, and any personnel in regular contact with the study site remained blinded to all patient randomization assignments.

Lipid results from blood samples collected after the randomization visit were not communicated to the sites, and the sponsor's operational team did not have access to these laboratory results until after the completion of the double-blind treatment period and the first step analysis.

Blinded investigational study drug kits coded with a medication numbering system were used. In order to maintain the blind, lists linking these codes with product lot numbers were not accessible to individuals involved in study conduct.

Anti-drug antibody (ADA) was not communicated to the sites, and the sponsor's operational team did not have access to results associated with patient identification until after the database lock after completion of the double-blind treatment period.

While the study was ongoing, it was anticipated that unblinded data, after the first step analysis (conducted as soon as all patients were randomized and all data through week 12 (double-blind period) were collected and validated; this consisted of the final analysis of the double-blind primary and secondary efficacy endpoints), would be submitted to health authorities. Sponsor representatives who conducted and reviewed such data analyses for submission to the health authorities were not part of the study operational team from that point forward, and patient level results were not provided to the study sites.

### Emergency Unblinding

Unblinding of treatment assignment for a patient could become necessary due to a medical emergency or any other significant medical event (*e.g*., pregnancy). If unblinding was required, only the investigator made the decision to unblind the treatment assignment, and only the affected patient was unblinded.

Treatment assignment was not provided to site personnel at any time during the conduct of the study, except in the case of a true emergency. In the event that there was no study pharmacist, the individual at the site fulfilling that role was the only unblinded member of the site personnel.

### Treatment Logistics and Accountability

As to packaging, labeling, and storage, a medication numbering system was used to label blinded investigational study drug. Lists linking medication numbers with product lot numbers were maintained by the groups (or companies) responsible for the investigational study drug packaging. In order to maintain the blind, these lists were not accessible to individuals involved in study conduct. Training kits containing 1 placebo prefilled pen were provided to the sites for patient/caregiver injection training that was performed before randomization during the screening period or at the baseline visit. A second placebo prefilled pen could be used before randomization if the patient/caregiver required additional injection training. Study drug was refrigerated at the site at a temperature of 2°C to 8°C. Storage temperature was logged. Detailed storage instructions were provided in the study manual.

As to supply and disposition of treatments, study drug was shipped at a temperature of 2°C to 8°C to the investigator or designee at regular intervals or as needed during the study. At specified time points during the study (*e.g*., interim site monitoring visits), at the site close-out visit, and following drug reconciliation and documentation by the site monitor, all opened and unopened investigational study drug were to be returned to the sponsor or designee. The investigational study drug was dispensed to each patient. The investigational study drug was stored, prepared, and administered by the patient/caregiver according to instructions provided to each patient/caregiver.

As to treatment accountability, all drug accountability records were kept current. The investigator had to be able to account for all opened and unopened investigational study drug. These records were to contain the dates, quantity, and study medication dispensed to each patient, returned from each patient (if applicable), and disposed of at the site or returned to the sponsor or designee.

Patients completed a dosing log to document compliance with the investigational study drug administration. Measures taken to ensure and document the investigational study drug accountability and compliance were:
i) the investigator or designee obtained via IVRS/IWRS the treatment kit number(s) and dispensed the treatment kit(s) to the patient,
ii) accountability was verified during the investigational study drug kit re-supply visits only; the used and unused kit(s) were to be brought to these visits for accountability purposes,
iii) all kits, including used and unused kits, were to be returned by the patient at the designated visit; an unused kit contained all of the unused prefilled pens; a used kit was one from which the patient had removed 1 or more prefilled pens; a used prefilled pen was one that had been removed from the kit with the intention of administration, including those injections that had been partially or fully injected; the patient was to discard all used prefilled pens into the sharps container and never put used prefilled pens back into the used kit,
iv) all sharps containers were to be returned to the site by the patient,
v) the investigator/study coordinator entered data in the appropriate CRF pages, according to data recorded in the treatment log form, and
vi) the monitor checked the data consistency among CRF pages, treatment log form, and returned unused prefilled pens of a corresponding kit.

All treatments kits were retrieved by the sponsor. A detailed treatment log of the returned investigational study drug was established with the investigator or designee and countersigned by the investigator and the monitoring team.

As to treatment compliance, all drug compliance records were to be kept current and made available for inspection by the sponsor and regulatory agency inspectors. Patients completed a dosing log to document compliance with the investigational study drug administration.

### Concomitant Medications

Concomitant medications were to be kept to a minimum during the study. If considered necessary for the patient's welfare and unlikely to interfere with the investigational study drug, concomitant medications (other than those that were prohibited during the study) could be given at the discretion of the investigator, at a stable dose when possible. Any treatments administered from the time of informed consent/assent to the final study visit were considered concomitant medications. This included medications that were started before the study and were ongoing during the study.

Prohibited medications and procedures included: a) use of a PCSK9 inhibitor within 10 weeks from the screening visit, b) initiation of or changes to the LDL apheresis schedule and/or settings (if applicable) or background medical LMT from the initial screening visit until the end of study visit, c) use of continuous estrogen or testosterone hormone replacement therapy, unless the regimen had been stable in the past 6 weeks prior to the screening visit, and d) chronic use of systemic corticosteroids, unless on a stable regimen of 10 mg daily prednisone equivalent or less for at least 6 weeks prior to randomization. As a note, topical, intra articular, nasal, inhaled and ophthalmic steroid therapies were not considered as 'systemic' and were allowed.

Permitted medications and procedures included lipid modifying therapies, nutraceuticals, and over-the-counter therapies that may affect lipids, but only if they had been used at a stable dose and regimen for at least 4 weeks (6 months for mipomersen, 12 weeks for the maximum tolerated dose of lomitapide) before the screening visit. The dose and regimen had to remain stable until the end of study visit. Low-density lipoprotein apheresis was allowed only if the schedule/settings had been stable for at least 8 weeks before the screening visit and remained stable until the end of study visit.

The lipid-modifying therapy (LMT) history of the patients in the instant study are summarized in the following table:

**Table 2: LMT History - Randomized Population**

| | | | | Placebo (N=24) | Alirocumab 150 Q2W (N=45) | All (N=69) |
|---|---|---|---|---|---|---|
| Overall on a maximally tolerated LMT per investigator [n(%)] | | | | | | |
| | Yes | | | 22 (91.7%) | 44 (97.8%) | 66 (95.7%) |
| | No | | | 2 (8.3%) | 1 (2.2%) | 3 (4.3%) |
| | | | | | | |

| On statin at screening [n(%)] | | | | | | |
|---|---|---|---|---|---|---|
| | Yes | | | 23 (95.8%) | 44 (97.8%) | 67 (97.1%) |
| | No | | | 1 (4.2%) | 1 (2.2%) | 2 (2.9%) |
| | | | | | | |

| On High intensity HMG COA inhibitor (statin) | | | | | | |
|---|---|---|---|---|---|---|
| | Yes | | | 23 (95.8%) | 44 (97.8%) | 67 (97.1%) |
| | | Patient on the maximum tolerated dose | | 23 (95.8%) | 42 (93.3%) | 65 (94.2%) |
| | No | | | 1 (4.2%) | 1 (2.2%) | 2 (2.9%) |
| | | Reason | | | | |
| | | | Intolerance - experienced muscle symptoms | 0 | 1 (2.2%) | 1 (1.4%) |
| | | | Intolerance - other symptoms | 0 | 0 | 0 |

| | | | Placebo (N=24) | Alirocumab 150 Q2W (N=45) | All (N=69) | |
|---|---|---|---|---|---|---|
| | | Concern about drug interaction | 0 | 0 | 0 | |
| | | Concern for potential adverse effects due to use (such as cognitive impairment) | 0 | 0 | 0 | |
| | | Lack of efficacy | 0 | 0 | 0 | |
| | | Not an approved medication | 0 | 0 | 0 | |
| | | Regional practices/local prescribing information | 0 | 0 | 0 | |
| | | Lack of access | 0 | 0 | 0 | |
| | | Other | 1 (4.2%) | 0 | 1 (1.4%) | |
| | | | | | | |

| History of down titration of statin dose due to tolerability | | | | | | |
|---|---|---|---|---|---|---|
| | Yes | | 4 (16.7%) | 12 (26.7%) | 16 (23.2%) | |
| | No | | 20 (83.3%) | 33 (73.3%) | 53 (76.8%) | |
| | | | | | | |

| History of change to different statin due to tolerability | | | | | | |
|---|---|---|---|---|---|---|
| | Yes | | 4 (16.7%) | 9 (200%) | 13 (18.8%) | |
| | No | | 20 (83.3%) | 36 (800%) | 56 (81.2%) | |
| | | | | | | |

| LLT at screening^{a} [n (%)] | | | | | | |
|---|---|---|---|---|---|---|
| | High Intensity HMG COA inhibitor (Statin) | | 23 (95.8%) | 44 (97.8%) | 67 (97.1%) | |
| | Fibrates | | 1 (4.2%) | 0 | 1 (1.4%) | |
| | Bile acid sequestrant | | 2 (8.3%) | 3 (6.7%) | 5 (7.2%) | |
| | Cholesterol absorption inhibitor | | 3 (12.5%) | 6 (13.3%) | 9 (130%) | |
| | Nicotinic acid and derivatives (Niacin) | | 1 (4.2%) | 1 (2.2%) | 2 (2.9%) | |
| | Omega 3 fatty acids ( > 1000 mg/day) | | 0 | 6 (13.3%) | 6 (8.7%) | |
| | Ezetimibe | | 19 (79.2%) | 31 (68.9%) | 50 (72.5%) | |
| | PCSK9 inhibitor | | 0 | 0 | 0 | |
| | Probucol [1] | | 0 | 0 | 0 | |
| | Apheresis | | 4 (16.7%) | 6 (13.3%) | 10 (14.5%) | |
| | Lomitapide | | 3 (12.5%) | 7 (15.6%) | 10 (14.5%) | |
| | Mipomersen | | 0 | 0 | 0 | |
| ^{a} patient can be counted in several categories. | | | | | | |

The lipid efficacy parameters at baseline of the patients in the instant study are summarized in the following table (quantitative summary in conventional units - randomized population):

**Table 3**

| | | Placebo (N=24) | Alirocumab 150 Q2W (N=45) | All (N=69) | P-Value vs. Placebo |
|---|---|---|---|---|---|
| LDL-C (mg/dL) | | | | | |
| | Number | 24 | 45 | 69 | 0.2338 |
| | Mean (SD) | 259.6 (175.75) | 295.0 (154.59) | 282.7 (161.86) | |
| | Median | 232.0 | 248.0 | 240.0 | |
| | Q1 : Q3 | 152.5 : 296.5 | 186.0 : 366.0 | 183.0 : 346.0 | |
| | Min : Max | 86 : 896 | 78 : 767 | 78 : 896 | |
| | | | | | |

| Non-HDL-C (mg/dL) | | | | | |
|---|---|---|---|---|---|
| | Number | 24 | 45 | 69 | 0.2313 |
| | Mean (SD) | 282.0 (177.41) | 320.5 (160.36) | 307.1 (166.22) | |
| | Median | 259.0 | 275.0 | 275.0 | |
| | Q1 : Q3 | 167.0 : 319.0 | 203.0 : 416.0 | 195.0 : 364.0 | |
| | Min : Max | 102: 928 | 92 : 791 | 92 : 928 | |
| | | | | | |

| Total-C (mg/dL) | | | | | |
|---|---|---|---|---|---|
| | Number | 24 | 45 | 69 | 0.1966 |
| | Mean (SD) | 325.1 (171.57) | 364.3 (157.30) | 350.7 (162.24) | |
| | Median | 305.5 | 326.0 | 315.0 | |
| | Q1 : Q3 | 209.0 : 362.0 | 253.0 : 444.0 | 235.0 : 400.0 | |
| | Min : Max | 155 : 947 | 162 : 830 | 155 : 947 | |
| | | | | | |

| HDL-C (mg/dL) | | | | | |
|---|---|---|---|---|---|
| | Number | 24 | 45 | 69 | 0.7526 |
| | Mean (SD) | 43.2 (11.96) | 43.8 (1478) | 43.6 (1378) | |
| | Median | 46.0 | 40.0 | 400 | |
| | Q1 : Q3 | 350 : 53.5 | 32.0 : 500 | 32.0 : 53.0 | |
| | Min : Max | 19 : 60 | 24 : 95 | 19 : 95 | |
| | | | | | |

| Fasting TGs (mg/dL) | | | | | |
|---|---|---|---|---|---|
| | Number | 24 | 45 | 69 | 0.1403 |
| | Mean (SD) | 111.7 (77.97) | 128.0 (74.34) | 122.3 (75.45) | |
| | Median | 80.5 | 110.0 | 97.0 | |
| | Q1 : Q3 | 61.0 : 128.5 | 79.0 : 160.0 | 72.0 : 148.0 | |
| | Min : Max | 41 : 305 | 25 : 346 | 25 : 346 | |
| | | | | | |

| Lp(a) (mg/dL) | | | | | |
|---|---|---|---|---|---|
| | Number | 24 | 45 | 69 | 0.7719 |
| | Mean (SD) | 400 (36.41) | 42.9 (36.34) | 41.9 (36.12) | |
| | Median | 32.5 | 36.0 | 36.0 | |
| | Q1 : Q3 | 12.0 : 52.5 | 10.0 : 68.0 | 10.0 : 65.0 | |
| | Min : Max | 4 : 144 | 4 : 157 | 4 : 157 | |

| | | Placebo (N=24) | Alirocumab 150 Q2W (N=45) | All (N=69) | P-Value vs. Placebo |
|---|---|---|---|---|---|
| Apo-B (mg/dL) | | | | | |
| | Number | 24 | 45 | 69 | 0.2675 |
| | Mean (SD) | 175.0 (95.12) | 193.3 (8759) | 186.9 (90.01) | |
| | Median | 161.0 | 178.0 | 165.0 | |
| | Q1 : Q3 | 110.0 : 202.0 | 132.0 : 232.0 | 124.0 : 220.0 | |
| | Min : Max | 82 : 525 | 66 : 468 | 66 : 525 | |

| Apo-A1 (mg/dL) | | | | | |
|---|---|---|---|---|---|
| | Number | 24 | 45 | 69 | 0.7913 |
| | Mean (SD) | 124.8 (24.59) | 125.6 (28.57) | 125.3 (27.07) | |
| | Median | 124.5 | 117.0 | 118.0 | |
| | Q1 : Q3 | 108.0 : 144.0 | 106.0 : 143.0 | 108.0 : 144.0 | |
| | Min : Max | 67 : 164 | 80 : 206 | 67 : 206 | |
| | | | | | |

| Apo-B/Apo-A1 (ratio) | | | | | |
|---|---|---|---|---|---|
| | Number | 24 | 45 | 69 | 0.2077 |
| | Mean (SD) | 1.590 (1.4746) | 1.635 (0.8693) | 1.619 (1.1067) | |
| | Median | 1.235 | 1.400 | 1.380 | |
| | Q1 : Q3 | 0.860: 1.630 | 1.020 : 1.990 | 0.970 : 1.930 | |
| | Min : Max | 0.54 : 7.84 | 0.38 : 4.46 | 0.38 : 7.84 | |
| Note: p-values comparing baseline data between treatment groups are provided for descriptive purpose, as a screening tool, using the asymptotic one-way ANOVA test for Wilcoxon scores (Kruskal-Wallis test). | | | | | |

The lipid efficacy parameters at baseline of the patients in the instant study are summarized in the following table (qualitative summary- randomized population):

**Table 4**

| | | Placebo (N=24) | Alirocumab 150 Q2W (N=45) | All (N=69) | P-Value vs. Placebo | |
|---|---|---|---|---|---|---|
| LDL-C | | | | | | |
| | Number | 24 | 45 | 69 | 0.2678 | |
| | <70 mg/dL / <1.81 mmol/L | 0 | 0 | 0 | | |
| | >=70 to <100 mg/dL / >=1.81 to <2.59 mmol/L | 4 (16.7%) | 1 (2.2%) | 5 (7.2%) | | |
| | >=100 to <130 mg/dL / >=2.59 to <3.37 mmol/L | 1 (4.2%) | 4 (8.9%) | 5 (7.2%) | | |
| | >=130 to <160 mg/dL / >=3.37 to <4.14 mmol/L | 1 (4.2%) | 2 (4.4%) | 3 (4.3%) | | |
| | >=160 to <190 mg/dL / >=4.14 to <4.91 mmol/L | 3 (12.5%) | 5 (11.1%) | 8 (11.6%) | | |
| | >=190 mg/dL / >=4.91 mmol/L | 15 (62.5%) | 33 (73.3%) | 48 (69.6%) | | |
| | | | | | | |

| HDL-C | | | | | | |
|---|---|---|---|---|---|---|
| | Number | 24 | 45 | 69 | 0.6192 | |
| | <40 mg/dL / <1.04 mmol/L | 10 (41.7%) | 22 (48.9%) | 32 (46.4%) | | |
| | >=40 mg/dL / >=1.04 mmol/L | 14 (58.3%) | 23 (51.1%) | 37 (53.6%) | | |
| | | | | | | |

| Fasting TGs | | | | | | |
|---|---|---|---|---|---|---|
| | Number | 24 | 45 | 69 | 0.7709 | |
| | <150 mg/dL / <1.7 mmol/L | 19 (79.2%) | 33 (73.3%) | 52 (75.4%) | | |
| | >=150 mg/dL / >=1.7 mmol/L | 5 (20.8%) | 12 (26.7%) | 17 (24.6%) | | |

| | | | Placebo (N=24) | Alirocumab 150 Q2W (N=45) | All (N=69) | P-Value vs. Placebo |
|---|---|---|---|---|---|---|
| | | >=150 to <200 mg/dL / >=1.7 to <2.3 mmol/L | 2 (8.3%) | 5 (11.1%) | 7 (10.1%) | |
| | | >=200 mg/dL / >=2.3 mmol/L | 3 (12.5%) | 7 (15.6%) | 10 (14.5%) | |
| | | | | | | |

| Lp(a) | | | | | | |
|---|---|---|---|---|---|---|
| | Number | | 24 | 45 | 69 | 0.8006 |
| | <30 mg/dL / <0.3 g/L | | 12 (500%) | 20 (44.4%) | 32 (46.4%) | |
| | >=30 mg/dL / >=0.3 g/L | | 12 (500%) | 25 (55.6%) | 37 (53.6%) | |
| | | >=30 to <50 mg/dL / >=0.3 to <0.5 g/L | 5 (20.8%) | 10 (22.2%) | 15 (21.7%) | |
| | | >=50 mg/dL / >=0.5 g/L | 7 (29.2%) | 15 (33.3%) | 22 (31.9%) | |
| Note: p-values comparing baseline data between treatment groups are provided for descriptive purpose, as a screening tool, using Fisher exact test. | | | | | | |

The summary of the patients in the instant study by mutation status are summarized in the following table (randomized population):

**Table 5**

| | | | Placebo (N=24) | Alirocumab 150 Q2W (N=45) | All (N=69) | P-Value vs. Placebo |
|---|---|---|---|---|---|---|
| Genotype State | | | | | | |
| | Homozygous | | 11 (45.8%) | 19 (42.2%) | 30 (43.5%) | 0.6160 |
| | Compound Heterozygous | | 7 (29.2%) | 11 (24.4%) | 18 (26.1%) | |
| | Double Heterozygous | | 0 | 4 (8.9%) | 4 (5.8%) | |
| | Other (Heterozygous, undetermined, or no mutation) | | 6 (25.0%) | 11 (24.4%) | 17 (24.6%) | |
| | | | | | | |
| | Homozygous (LDLR) | | 10 (41.7%) | 18 (40.0%) | 28 (40.6%) | |
| | | Defective/Defective | 10 (41.7%) | 16 (35.6%) | 26 (37.7%) | |
| | | Negative/Negative | 0 | 2 (4.4%) | 2 (2.9%) | |
| | | | | | | |
| | Homozygous (LDLRAP1) | | 0 | 1 (2.2%) | 1 (1.4%) | |
| | | Negative/Negative | 0 | 1 (2.2%) | 1 (1.4%) | |
| | | | | | | |
| | Homozygous (PCSK9) | | 1 (4.2%) | 0 | 1 (1.4%) | |
| | | Defective/Defective | 1 (4.2%) | 0 | 1 (1.4%) | |
| | | | | | | |
| | Compound Heterozygous (LDLR) | | 7 (29.2%) | 11 (24.4%) | 18 (26.1%) | |
| | | Defective/Defective | 4 (16.7%) | 7 (15.6%) | 11 (15.9%) | |
| | | Defective/Negative | 3 (12.5%) | 4 (8.9%) | 7 (10.1%) | |
| | | Negative/Negative | 0 | 0 | 0 | |
| | | | | | | |
| | Double Heterozygous (LDLR and APOB and PCSK9) | | 0 | 4 (8.9%) | 4 (5.8%) | |
| | | Defective (LDLR)/Negative (APOB) | 0 | 1 (2.2%) | 1 (1.4%) | |
| | | Defective (LDLR)/Defective (APOB) | 0 | 1 (2.2%) | 1 (1.4%) | |
| | | Defective (LDLR)/Defective (PCSK9) | 0 | 1 (2.2%) | 1 (1.4%) | |
| | | Negative (LDLR)/Defective (PCSK9) | 0 | 1 (2.2%) | 1 (1.4%) | |
| Note: p-values comparing baseline data between treatment groups are provided for descriptive purpose, as a screening tool, using Fisher exact test. | | | | | | |

The summary of the patients in the instant study by mutation status are summarized in the following table (Null/Null vs Not Null/Null): LDLR Activity < 2% - Randomized Population:

**Table 6**

| | | | Placebo (N=24) | Alirocumab 150 Q2W (N=45) | All (N=69) | P-Value vs. Placebo |
|---|---|---|---|---|---|---|
| Genotype State | | | | | | |
| | Homozygous (LDLR) | | | | | |
| | | Null/Null | 1 (4.2%) | 4 (8.9%) | 5 (7.2%) | 0.6264 |
| | | Not Null/Null | 9 (37.5%) | 14 (31.1%) | 23 (33.3%) | |
| | | | | | | |
| | Homozyoous (LDLRAP1) | | | | | |
| | | Null/Null | 0 | 0 | 0 | |
| | | | | | | |
| | Compound Heterozygous (LDLR) | | | | | |
| | | Not Null/Null | 7 (29.2%) | 11 (24.4%) | 18 (26.1%) | |
| | | | | | | |
| Null/Null is defined as LDLR activity < 2% | | | | | | |
| Note: p-values comparing baseline data between treatment groups are provided for descriptive purpose, as a screening tool, using Fisher exact test. | | | | | | |

### Study Schedule of Events and Procedures

The study assessments and procedures are presented by study period in Table 7, below.

**Table 7**

| | **Optional Run-in** | **Screening Period** | **Double-Blind Treatment Period** | | | | | **Open-label Treatment Period** | | **Follow-up¹⁰** |
|---|---|---|---|---|---|---|---|---|---|---|
| **Study Procedure** | **Run-in Visit 1a** | **Screening Visit 1** | **Baseline Visit 2** | **Visit 3** | **Visit 4** | **Visit 5** | **End of Double-Blind Treatment Visit 6** | **Visit 7** | **End of Open-Label Treatment Visit 8** | **End of Study Phone Visit 9** |
| Day | -42 to -14 | -14 to -1 | 1 (±1) | 29(± 5/±1 ⁹) | 57(± 5/±1 ⁹) | 71(± 5/±1⁹ ) | 85(±3/±1 ⁹) | 127( ±7) | 169(±3/± 1⁹) | 225(±5) |
| Week | -6 to -2 | -2 to -1 | 0 | 4 | 8 | 10 | 12 | 18 | 24 | 32 |

| **Screening/Baseline:** | | | | | | | | | | |
|---|---|---|---|---|---|---|---|---|---|---|
| Inclusion/ Exclusion | X | X | | | | | | | | |
| Informed Consent/ Assent¹ | X | X | | | | | | | | |
| Pharmacog enomics Consent/ Assent | | X | | | | | | | | |
| Medical/ Surgical | | X | | | | | | | | |

| | **Optional Run-in** | **Screening Period** | **Double-Blind Treatment Period** | | | | | **Open-label Treatment Period** | | **Follow-up¹⁰** |
|---|---|---|---|---|---|---|---|---|---|---|
| **Study Procedure** | **Run-in Visit 1a** | **Screening Visit 1** | **Baseline Visit 2** | **Visit 3** | **Visit 4** | **Visit 5** | **End of Double-Blind Treatment Visit 6** | **Visit 7** | **End of Open-Label Treatment Visit 8** | **End of Study Phone Visit 9** |
| History, Alcohol habits, Smoking habits | | | | | | | | | | |
| Demographics | | X | | | | | | | | |

| **Treatment:** | | | | | | | | | | |
|---|---|---|---|---|---|---|---|---|---|---|
| Injection training² | | X | X | | | | | | | |
| Investigational study drug kit dispensation | | | X | X | | | X | X | | |
| Administer SC double- blin d investigational study drug ³ | | | X | X | X | X | | | | |
| Administer open-label investigatio nal study drug³ | | | | | | | X | X | | |
| Kit return | | | | X | X | | | X | X | |
| Review of dosing log | | | | X | X | | | X | X | |
| Concomitant medications | X | X | X | X | X | X | X | X | X | X |

| **Efficacy:** | | | | | | | | | | |
|---|---|---|---|---|---|---|---|---|---|---|
| Lipid panel⁴ | | X | X | X | X | | X | X | X | |
| Specialty lipid panel⁴ | | | X | X | X | | X | | X | |
| EQ-5D | | | X | | | | X | | X | |

| **Safety** | | | | | | | | | | |
|---|---|---|---|---|---|---|---|---|---|---|
| Adverse events | X | X | X | X | X | X | X | X | X | X |
| Physical examination | | X | | | | | X | | X | |
| Body weight | X | X | | | | | X | | X | |
| Vital signs | X | X | X | X | X | X | X | X | X | |

| | **Optional Run-in** | **Screening Period** | **Double-Blind Treatment Period** | | | | | **Open-label Treatment Period** | | **Follow-up¹⁰** |
|---|---|---|---|---|---|---|---|---|---|---|
| **Study Procedure** | **Run-in Visit 1a** | **Screening Visit 1** | **Baseline Visit 2** | **Visit 3** | **Visit 4** | **Visit 5** | **End of Double-Blind Treatment Visit 6** | **Visit 7** | **End of Open-Label Treatment Visit 8** | **End of Study Phone Visit 9** |
| Electrocardi ogram⁵ | | X | | | | | X | | X | |

| **Laboratory Testing:** | | | | | | | | | | |
|---|---|---|---|---|---|---|---|---|---|---|
| Hematology⁶ | | X | X | | X | | X | | X | |
| Blood chemistry⁶ | | X | X | | X | | X | | X | |
| Creatine Phosphokinase⁶ | | X | X | | X | | X | | X | |
| Hepatitis B surface antigen⁶ | | X | | | | | | | | |
| Hepatitis C antibody⁶ | | X | | | | | X | | X | |
| Serum pregnancy test | | X | | | | | | | | |
| Urine pregnancy test (done locally) | X | | | | | | X | | X | |
| Urinalysis | | X | X | | X | | X | | X | |
| TSH | | X | | | | | | | | |
| hs-CRP⁶ | | X | X | | | | X | | X | |
| Research samples⁶ | | | X | X | X | | X | | X | |
| Mandatory DNA collection for hoFH Genetic Testing⁷ | X | X | | | | | | | | |

| **PK/Drug Concentration and ADA Samples:** | | | | | | | | | | |
|---|---|---|---|---|---|---|---|---|---|---|
| PK Sample⁶ | | | X | X | X | X | X | | X | |
| ADA Sample⁶ | | | X | | | | X | | X | |
| Optional Genomic DNA Sample⁸ | | | X | | | | | | | |

The footnotes from Table 6, above, signify the following: 1. Informed consent/assent was obtained either at Visit 1a (for patients who needed the optional run-in time) or at Visit 1 (for the patients who did not need optional run-in). 2. Injection training was performed with the patient and/or caregiver during the screening period or at baseline using placebo. 3. After the investigational study drug administration (double-blind and open-label), patients needed to be monitored for 30 minutes. 4. Lipid panel consisted of: total-C, LDL-C, HDL-C, TG, non-HDL-C. Specialty lipid panel consisted of: ApoB, Apo A-1, ratio Apo B/Apo A-1, and Lp(a). Lipid panels were to be collected after an approximately 8 hour fast. 5. ECG was to be performed before blood samples were collected. 6. On days when a clinic visit coincided with a dosing day, all blood samples (including ADA samples) were collected immediately prior to LDL apheresis (if applicable) and before the investigational study drug administration, but after study assessments were performed. PK samples were also used for free and total PCSK9 analysis. 7. Sample was to be obtained prior to randomization and was used to determine hoFH mutation status. Patients on apheresis could collect this during visit 1a. 8. Optional DNA sample was to be collected on day 1; however, they could be collected at any visit during the course of the study. Genomic informed consent form (ICF) had to be signed prior to performing this assessment. 9. Visit window was ±3 days for patients not on apheresis and +1 day for patients on apheresis. Every attempt was to be made to ensure all samples were collected immediately prior to LDL apheresis. The timing between the baseline sample collection relative to the most recently completed LDL apheresis procedure was to match the timing of the week 12 sample collection relative to the most recently completed LDL apheresis procedure. Depending on the duration between the LDL apheresis procedure and sample collection, the visit window might not apply. 10. This visit was only for patients who did not participate in another lipid-lowering study.

With respect to early termination visits, if for any reason the patient refused to continue the study, the patient was to undergo an unscheduled visit as soon as possible with assessments normally planned at the end of double blind treatment visit if the patient was in the double-blind treatment period (week 12 visit assessments); if the patient was in the open-label treatment visit, then the end of the open-label treatment assessments were to be used (week 24 visit assessments). This visit was to take place within 5 days of treatment discontinuation, if possible. The patient was to be followed for at least 70 days from the last dose of study drug or up to recovery or stabilization of any AE to be followed-up as specified, whichever came last.

As to unscheduled visits, all attempts were to be made to keep the patients on the study schedule. Unscheduled visits could be necessary to repeat testing following abnormal laboratory results, for follow-up of AEs, or for any other reason, as warranted.

### Study Procedures

The following procedures were performed for the sole purpose of determining study eligibility or characterizing the baseline population: medical/surgical history, medication history, demographics, hepatitis B surface antigen, and serum pregnancy testing.

On day 1, after completion of study assessments, collection of blood samples, LDL apheresis (if applicable), the first dose of double-blind investigational study drug was administered. The patient was monitored at the clinical site for 30 minutes after the first dose. Subsequent doses of the investigational study drug were to be administered subcutaneously Q2W. Doses of the investigational study drug were to be administered at approximately the same time of day (based upon patient preference) throughout the study. After day 1, it was acceptable for dosing to fall within a window of ± 5 days, with the exception of the week 10 and week 22 doses, which had a ± 3 day dosing window for patients not on apheresis and a +1 day dosing window for patients on apheresis, since there were key efficacy assessments at week 12 and week 24.

In the event an injection was delayed by more than 7 days or completely missed, the patient was to return to the original schedule of the investigational study drug dosing without administering additional injections. If the delay was less than or equal to 7 days from the missed date, the patient was to administer the delayed injection and then resume the original dosing schedule. Site personnel provided the patient/caregiver with detailed instructions for transport, storage, preparation, and administration of the investigational study drug.

### Efficacy Procedures

Total cholesterol, HDL-C, TG, Apo B, Apo A-1, and Lp(a) were directly measured by the central laboratory. Low-density lipoprotein cholesterol was calculated using the Friedewald formula. If TG values exceeded 400 mg/dL (4.52 mmol/L), or if calculated LDL-C values were below 15 mg/dL, then the central lab reflexively measured LDL-C using the beta quantification method. Non-HDL-C was calculated by subtracting HDL-C from the Total C. The Apo B/Apo A-1 ratio was calculated.

Blood samples for the lipid panel and specialty lipid panel were collected at time points according to Table 7, above. What was included in the lipid panel and special lipid panel is described below.

### Quality of Life Procedures

EuroQol-5 Questionnaire: the EQ-5D is a standardized measure of health status developed by the EuroQol Group in order to provide a simple, generic measure of health for clinical and economic appraisal. The EQ-5D as a measure of health related quality of life, defines health in terms of 5 dimensions: mobility, self-care, usual activities, pain/discomfort, anxiety/depression. Each dimension has 3 ordinal levels of severity: "no problem" (1), "some problems" (2), "severe problems" (3). Overall health state is defined as a 5-digit number. Health states defined by the 5-dimensional classification can be converted into corresponding index scores that quantify health status, where 0 represents "death" and 1 represents "perfect health."

### Safety Procedures

Vital signs, including blood pressure and heart rate were collected at time points according to Table 7, above. A thorough and complete physical examination was performed at time points according to Table 7, above. Body weight was collected at time points according to Table 7, above. Care was to be taken to examine and assess any abnormalities that could be present, as indicated by the patient's medical history. Electrocardiogram was to be performed before blood was drawn during the visits requiring blood draws. A standard 12-lead ECG was performed with the patient in the supine position after resting quietly for 10 minutes at time points according to Table 7, above. Heart rate was recorded from the ventricular rate, and the PR, QRS, RR, and QT intervals were recorded. The ECG strips or reports were retained with the source.

All laboratory samples (including ADA samples) were collected after assessments were performed and before a dose of the investigational study drug was administered at visits that corresponded with a dosing day. Alcohol consumption within 48 hours or intense physical exercise within 24 hours preceding blood sampling was discouraged. Samples for laboratory testing were collected at time points according to Table 6, above, and analyzed by a central laboratory during the study. Lipid panel samples were collected after 8-hour fasting. Detailed instructions for blood sample collection were in the laboratory manual provided to study sites. Tests included:

### Lipid Panel and Specialty Lipid Panel

| | |
|---|---|
| Total cholesterol | Apo B |
| Triglyceride | Apo A-1 |
| Calculated LDL-C | Apo B/Apo A-1 ratio |
| HDL-C | Lp(a) |
| Non-HDL-C | |

### Blood Chemistry

| | | |
|---|---|---|
| Sodium | Total protein, serum | Total bilirubin |
| Potassium | Creatinine | Uric acid |
| Chloride | Blood urea nitrogen (BUN) | Creatine phosphokinase (CPK) |
| Carbon dioxide | Aspartate aminotransferase (AST) | |
| Calcium | Alanine aminotransferase (ALT) | |
| Glucose | Alkaline phosphatase | |
| Albumin | Lactate dehydrogenase (LDH) | |

### Hematology

| | |
|---|---|
| Hemoglobin | Differential: |
| Hematocrit | Neutrophils |
| Red blood cells (RBCs) | Lymphocytes |
| White blood cells (WBCs) | Monocytes |
| Red cell indices | Basophils |
| Platelet count | Eosinophils |

### Urinalysis

| | | |
|---|---|---|
| Color | Glucose | RBC |
| Clarity | Blood | Hyaline and other casts |
| pH | Bilirubin | Bacteria |
| Specific gravity | Leukocyte esterase | Epithelial cells |
| Ketones | Nitrite | Crystals |
| Protein | WBC | Yeast |

Other laboratory tests were performed as follows: pregnancy testing (serum and urine) was performed at time points according to Table 7, above, pregnancy testing (urine) was assessed locally at time points according to Table 7, above, samples for the liver panel (ALT, AST, alkaline phosphatase, and total bilirubin), high sensitivity C-reactive protein (hs-CRP) were collected at time points according to Table 7, above, and a sample for hepatitis B surface antigen, hepatitis C antibody, and TSH was collected at screening. Samples for hepatitis C antibody were collected at time points according to Table 6, above.

### Abnormal Laboratory Values and Laboratory Adverse Events

All laboratory values had to be reviewed by the investigator or authorized designee. Significantly abnormal test results that occurred after start of treatment had to be repeated to confirm the nature and degree of the abnormality. When necessary, appropriate ancillary investigations was to be initiated. If the abnormality failed to resolve or could not be explained by events or conditions unrelated to the study medication or its administration, the medical monitor was consulted. The clinical significance of an abnormal test value, within the context of the disease under study, was to be determined by the investigator.

The criteria for determining whether an abnormal objective test finding should be reported as an AE included: the test result we associated with accompanying symptoms, and/or the test result required additional diagnostic testing or medical/surgical intervention, and/or the test result led to a change in dosing (outside of protocol-stipulated dose adjustments), discontinuation from the study, significant additional concomitant drug treatment, or other therapy.

### Pharmacokinetic and Anti-Drug Antibody Procedures

Samples for drug concentration were collected at time points listed in Table 7, above. Any unused samples could be used for exploratory biomarker research.

Samples for anti-drug antibody (ADA) assessment were collected at time points listed in Table 6, above. At visits that took place on dosing days, all samples for ADA assessments were collected before a dose of the investigational study drug was administered. To maintain the blind of the study, ADA samples were collected from all patients, including those who received only placebo. Any unused samples could be used for exploratory biomarker research.

### hoFH Genetic Testing

A sample was collected for mandatory hoFH genetic testing to characterize the mutation status of each patient as listed in Table 7, above.

### Research Samples

Samples for exploratory research were collected as allowed by local regulations to study PCSK9 levels, PCSK9 function, effects of PCSK9 inhibition with a monoclonal antibody, and mechanisms of hyperlipidemia and heart disease. Research sampling was collected at time points according to Table 7, above. Research samples were coded to maintain patient confidentiality.

### Biomarker Procedures

Biomarker samples were collected at time points according to Table 7, above, as part of the Research Samples. Biomarker measurements were performed in matrix, for example, serum samples to determine effects on biomarkers of indication or relevant physiological and pathogenic processes. The biomarkers studied were ones believed to be relevant to the pathophysiology of indication target engagement, mechanism of action, and possible toxicities. Biomarkers studied could include, but were not limited to, PCSK9.

### Adverse Events

An adverse event (AE) is any untoward medical occurrence in a patient administered an investigational study drug, which may or may not have a causal relationship with the investigational study drug. Therefore, an AE is any unfavorable and unintended sign (including abnormal laboratory finding), symptom, or disease temporally associated with the use of the investigational study drug, whether or not considered related to the investigational study drug. An AE also includes any worsening (*i.e*., any clinically significant change in frequency and/or intensity) of a pre-existing condition that is temporally associated with the use of the investigational study drug.

An adverse event of special interest (serious or non-serious) is one of scientific and medical concern specific to the sponsor's product or program, for which ongoing monitoring and rapid communication by the investigator to the sponsor can be appropriate. Adverse events of special interest for this study include the following: a) increase in ALT: ALT ≥3 x ULN (if baseline ALT <ULN), or ALT ≥2 times the baseline value (if baseline ALT ≥ ULN), b) allergic events and/or local injection site reactions that require consultation with another physician for further evaluation, c) pregnancy, d) symptomatic overdose with investigational medicinal product, e) neurologic events that require additional examinations/procedures and/or referral to a specialist, neurocognitive events, f) cataracts, g) new onset of diabetes (where the definition of new onset of diabetes (NOD) is: Type 1 or type 2 diabetes TEAE, and/or h) at least 2 values of HbA1c ≥6.5% during the TEAE period (NOTE: for patients with only a single measurement available during the TEAE period, a single value ≥6.5% will be considered and qualify the patient as NOD by default; for patients with several HbA1c measurements but only with the last one ≥6.5%, this single value ≥6.5% will be considered and qualify the patient as NOD by default), and/or i) at least 2 values of fasting plasma glucose (FPG) ≥126 mg/dL (7.0 mmol/L) (NOTE: for patients with only a single measurement available during the TEAE period, a single value ≥126 mg/dL (7.0 mmol/L) will NOT be considered and will NOT qualify the patient as NOD; for patients with several FPG measurements but only with the last one ≥126 mg/dL (7.0 mmol/L), this single value ≥ 126 mg/dL (7.0 mmol/L) will NOT be considered and will NOT qualify the patient as NOD).

### Serious Adverse Events

An SAE (serious adverse event) is any untoward medical occurrence that at any dose: a) results in death - includes all deaths, even those that appear to be completely unrelated to the investigational study drug (*e.g*., a car accident in which a patient is a passenger), b) is life-threatening - in the view of the investigator, the patient is at immediate risk of death at the time of the event; this does not include an AE that had it occurred in a more severe form, might have caused death, c) requires in-patient hospitalization or prolongation of existing hospitalization (where in patient hospitalization is defined as admission to a hospital or an emergency room for longer than 24 hours; prolongation of existing hospitalization is defined as a hospital stay that is longer than was originally anticipated for the event, or is prolonged due to the development of a new AE as determined by the investigator or treating physician), d) results in persistent or significant disability/incapacity (substantial disruption of one's ability to conduct normal life functions), e) is a congenital anomaly/birth defect, and/or f) is an important medical event - important medical events may not be immediately life-threatening or result in death or hospitalization, but may jeopardize the patient or may require intervention to prevent one of the other serious outcomes listed above (*e.g*., intensive treatment in an emergency room or at home for allergic bronchospasm; blood dyscrasias or convulsions that do not result in hospitalization; or development of drug dependency or drug abuse).

Thus, the instantly disclosed randomized, double-blind, placebo-controlled, parallel-group, phase 3 study evaluated the efficacy and safety of PCSK9 inhibitor, alirocumab 150 mg subcutaneous, every 2 weeks in reduction of LDL-C compared with placebo after 12 weeks of treatment in adult patients with hoFH. Secondary objectives included evaluation of 1) other lipid parameters (ie, apolipoprotein B [Apo B], non-high-density lipoprotein cholesterol [non-HDL-C], total-cholesterol [TC], proportion of patients with 15%, 30%, and 50% LDL-C reductions, lipoprotein(a) [Lp(a)], HDL-C, triglycerides [TG], Apo A-1); 2) the safety and tolerability of alirocumab; 3) the pharmacokinetics; 4) the potential development of anti-drug (alirocumab) antibodies. Finally, safety assessments included: adverse events (AEs), serious AEs, deaths, discontinuations due to AE.

### RESULTS

### Primary efficacy analysis in the ITT population

The primary efficacy analysis showed a statistically significant decrease in percent change from baseline LDL-C at Week 12 for the alirocumab treatment group (LSmean = -26.9%) as compared to the placebo group (LSmean = +8.6%). The LS mean difference between the alirocumab-treated patients and the placebo patients is -35.6% (p<0.0001). The alirocumab LSmean reductions in percent change LDL-C from baseline could be seen as early as visit week 4, and alirocumab benefit was subsequently maintained throughout the 12 week double-blind treatment period.

The percent change from baseline in LDL-C at week 12 (MMRM - ITT Analysis - ITT Population) is shown in Table 8, below.

**Table 8**

| LDL Cholesterol | | Placebo (N=24) | Alirocumab 150 Q2W (N=45) |
|---|---|---|---|
| Baseline (mmol/L) | | | |
| | Number | 24 | 45 |
| | Mean (SD) | 6.723 (4.5524) | 7.640 (4.0040) |
| | Median | 6.010 | 6.420 |
| | Min : Max | 2.23 : 23.21 | 2.02 : 19.87 |
| Baseline (mg/dL) | | | |
| | Number | 24 | 45 |
| | Mean (SD) | 259.6 (175.75) | 295.0 (154.59) |
| | Median | 232.0 | 248.0 |
| | Min : Max | 86 : 896 | 78 : 767 |
| | | | |
| Week 12 percent change from baseline (%) | | | |
| | LS mean (SE) | 8.6 (6.3) | -26.9 (4.6) |
| | LS mean difference (SE) vs Placebo | | -35.6 (7.8) |
| | 95% CI | | ( -51.2 to -19.9) |
| | p-value vs Placebo | | <.0001 |
| | | | |
| Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline LDL-C value and baseline value by time-point interaction. | | | |
| MMRM model and baseline description run on patients with a baseline value and a post-baseline value in at least one of the analysis windows used in the model. | | | |

The LDL-C LS Mean (+/-SE) percent change from baseline for the double-blind period: time profile (ITT analysis - ITT population) (data not shown) shows a statistically significant decrease in % change in LDL-C from baseline to week 12. For alirocumab, LSmean vs. baseline is -26.9% (in other words, a decrease in LDL-C). For placebo, LSmean vs. baseline is 8.6% (in other words, an increase in LDL-C).

The number of patients at the different timepoints (through week 12) were as follows:

### Number of patients

| Treatment | baseline | week 4 | week 8 | week 12 |
|---|---|---|---|---|
| Placebo | 24 | 24 | 24 | 24 |
| Alirocumab | 45 | 43 | 45 | 44 |

The LDL-C efficacy in the instant hoFH study is summarized as follows:

| Timepoint | placebo (24 patients) | alirocumab (45 patients) | placebo adjusted difference |
|---|---|---|---|
| Wk 4 | -0.7% | -31.9% | -31.3% |
| Wk 8 | 5.2% | -27.9% | -33.1% |
| Wk 12 | 8.6% | -26.9% | -35.6% |

### Key secondary efficacy variables

For easy reference, the following table (Table 9) summarizes analysis results on all key secondary endpoints in the hierarchical order for statistical testing at the 0.05 significance level. The instant study achieved statistically significant results in favor of the alirocumab-treated patients for the top 7 key efficacy endpoints. Statistical hypothesis testing terminates at the 8th endpoint of "Percent change from baseline in HDL-C at WK12" (p = 0.3541). Nominal p-values for the two remaining endpoints of "Percent change from baseline in fasting TG at WK12" and "Percent change from baseline in Apo A-1 at WK12" are provided for descriptive purposes.

**Table 9**

| **Order** | **Endpoint/Analysis** | **Placebo Result** | **Alirocumab Result** | **Comparison** | **P-value** |
|---|---|---|---|---|---|
| 1 | Percent change from baseline in Apo B at WK12 | LS mean: 7.2% | LS mean: - 22.5% | Diff: -29.8% | <.0001 |
| 2 | Percent change from baseline in non-HDL-C at WK12 | LS mean: 8.0% | LS mean: - 24.8% | Diff: -32.9% | <.0001 |
| 3 | Percent change from baseline in total cholesterol at WK12 | LS mean: 6.6% | LS mean: - 19.8% | Diff: -26.5% | <.0001 |
| 4 | ≥ 15% reduction in LDL-C at WK12 | Proportion: 12.5% | Proportion: 61.9% | Odds Ratio: 12.2 | 0.0004 |
| 5 | ≥ 30% reduction in LDL-C at WK12 | Proportion: 4.2% | Proportion: 57.1% | Odds Ratio: 36.5 | 0.0010 |
| 6 | Percent change from baseline in Lp(a) at WK12 | LS mean: 8.8% | LS mean: - 19.6% | Diff: -28.4% | <.0001 |
| 7 | ≥ 50% reduction in LDL-C at WK12 | Proportion: 0% | Proportion: 26.7% | Odds Ratio*: 17.7 | 0.0017 |
| 8 | Percent change from baseline in HDL-C at WK12 | LS mean: 2.7% | LS mean: 6.3% | Diff: 3.6% | 0.3541 |
| 9 | Percent change from baseline in fasting TG at WK12 | LS mean: 3.9% | LS mean: -7.4% | Diff: -11.3% | 0.1112 |
| 10 | Percent change from baseline in Apo A-1 at WK12 | LS mean: 1.4% | LS mean: 5.0% | Diff: 3.6% | 0.3212 |

More detailed efficacy endpoint statistical results are presented in the following tables. The percent change from baseline in Apo B at week 12 (MMRM - ITT Analysis - ITT Population) results are provided in Table 10, below.

**Table 10**

| Apolipoprotein B | | Placebo (N=24) | Alirocumab 150 Q2W (N=45) |
|---|---|---|---|
| Baseline (g/L) | | | |
| | Number | 24 | 45 |
| | Mean (SD) | 1.750 (0.9512) | 1.933 (0.8759) |
| | Median | 1.610 | 1.780 |
| | Min : Max | 0.82 : 5.25 | 0.66 : 4.68 |
| | | | |

| Baseline (mg/dL) | | | |
|---|---|---|---|
| | Number | 24 | 45 |
| | Mean (SD) | 175.0 (95.12) | 193.3 (87.59) |
| | Median | 161.0 | 178.0 |
| | Min : Max | 82 : 525 | 66 : 468 |
| | | | |

| Week 12 percent change from baseline (%) | | | |
|---|---|---|---|
| | LS mean (SE) | 7.2 (5.0) | -22.5 (3.7) |
| | LS mean difference (SE) vs Placebo | | -29.8 (6.3) |
| | 95% CI | | ( -42.3 to -17.3) |
| | p-value vs Placebo | | <.0001 |
| Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline ApoB value and baseline value by time-point interaction. | | | |

The percent change from baseline in non-HDL-C at week 12 (MMRM - ITT Analysis - ITT Population) results are provided in Table 11, below.

**Table 11**

| Non-HDL Cholesterol | | Placebo (N=24) | Alirocumab 150 Q2W (N=45) |
|---|---|---|---|
| Baseline (mmol/L) | | | |
| | Number | 24 | 45 |
| | Mean (SD) | 7.304 (4.5954) | 8.302 (4.1535) |
| | Median | 6.710 | 7.120 |
| | Min : Max | 2.64 : 24.04 | 2.38 : 20.49 |
| | | | |

| Baseline (mg/dL) | | | |
|---|---|---|---|
| | Number | 24 | 45 |
| | Mean (SD) | 282.0 (177.41) | 320.5 (160.36) |
| | Median | 259.0 | 275.0 |
| | Min : Max | 102: 928 | 92 : 791 |
| | | | |

| Non-HDL Cholesterol | | Placebo (N=24) | Alirocumab 150 Q2W (N=45) |
|---|---|---|---|
| Week 12 percent change from baseline (%) | | | |
| | LS mean (SE) | 8.0 (5.9) | -24.8 (4.3) |
| | LS mean difference (SE) vs Placebo | | -32.9 (7.4) |
| | 95% CI | | ( -47.6 to -18.2) |
| | p-value vs Placebo | | <.0001 |
| | | | |
| Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline non-HDL-C value and baseline value by time-point interaction. | | | |

The percent change from baseline in total cholesterol at week 12 (MMRM - ITT Analysis - ITT Population) results are provided in Table 12, below.

**Table 12**

| Total Cholesterol | | Placebo (N=24) | Alirocumab 150 Q2W (N=45) |
|---|---|---|---|
| Baseline (mmol/L) | | | |
| | Number | 24 | 45 |
| | Mean (SD) | 8.422 (4.4443) | 9.437 (4.0742) |
| | Median | 7.915 | 8.440 |
| | Min : Max | 4.01 : 24.53 | 4.20 : 21.50 |
| | | | |

| Baseline (mg/dL) | | | |
|---|---|---|---|
| | Number | 24 | 45 |
| | Mean (SD) | 325.1 (171.57) | 364.3 (157.30) |
| | Median | 305.5 | 326.0 |
| | Min : Max | 155 : 947 | 162 : 830 |
| | | | |

| Week 12 percent change from baseline (%) | | | |
|---|---|---|---|
| | LS mean (SE) | 6.6 (5.0) | -19.8 (3.7) |
| | LS mean difference (SE) vs Placebo | | -26.5 (6.2) |
| | 95% CI | | ( -38.9 to -14.0) |
| | p-value vs Placebo | | <.0001 |
| | | | |
| Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline total cholesterol value and baseline value by time-point interaction. | | | |

The percent change from baseline in Lp(a) at week 12 (multiple imputation followed by robust regression - ITT Analysis - ITT Population) results are provided in Table 13, below.

**Table 13**

| Lipoprotein-a | | Placebo (N=24) | Alirocumab 150 Q2W (N=45) |
|---|---|---|---|
| Baseline (g/L) | | | |
| | Combined estimate for mean (SE) | 0.400 (0.074) | 0.429 (0.054) |

| Baseline (mg/dL) | | | |
|---|---|---|---|
| | Combined estimate for mean (SE) | 40.0 (7.4) | 42.9 (5.4) |
| | | | |

| Week 12 percent change from baseline (%) | | | |
|---|---|---|---|
| | Combined estimate for adjusted mean (SE) | 8.8 (5.4) | -19.6 (4.0) |
| | Combined estimate for adjusted mean difference (SE) (Alirocumab vs Comparator) | | -28.4 (6.7) |
| | 95% CI | | (-41.5 to -15.2) |
| | p-value vs Placebo | | <.0001 |
| | | | |
| Note: The two-step multiple imputation procedure is used to address missing values in the randomized population (seeds=1628 and 3256; number of imputations=100 and 1 in the two steps respectively). In the first step, the monotone missing pattern is induced in the multiply-imputed data. In the second step, the missing data at subsequent visits are imputed using the regression method for continuous variables. | | | |
| Combined estimates and standard errors (SE) are obtained by combining adjusted means and SE from robust regression model analyses of the different imputed data sets. The robust regression models include the fixed categorical effect of treatment group and randomization strata as per IVRS and the continuous fixed covariate of baseline Lp(a) value. Rubin's formulae are used to combine means and SE. | | | |

The percent change from baseline in HDL-C at week 12 (MMRM - ITT Analysis - ITT Population) results are provided in Table 14, below.

**Table 14**

| HDL Cholesterol | | Placebo (N=24) | Alirocumab 150 Q2W (N=45) |
|---|---|---|---|
| Baseline (mmol/L) | | | |
| | Number | 24 | 45 |
| | Mean (SD) | 1.118 (0.3095) | 1.134 (0.3824) |
| | Median | 1.195 | 1.040 |
| | Min : Max | 0.49 : 1.55 | 0.62 : 2.46 |
| | | | |

| Baseline (mg/dL) | | | |
|---|---|---|---|
| | Number | 24 | 45 |
| | Mean (SD) | 43.2 (11.96) | 43.8 (14.78) |
| | Median | 46.0 | 40.0 |
| | Min : Max | 19 : 60 | 24 : 95 |
| | | | |

| Week 12 percent change from baseline (%) | | | |
|---|---|---|---|
| | LS mean (SE) | 2.7 (3.1) | 6.3 (2.3) |
| | LS mean difference (SE) vs Placebo | | 3.6 (3.8) |
| | 95% CI | | ( -4.1 to 11.3) |
| | p-value vs Placebo | | 0.3541 |
| | | | |
| Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline HDL-C value and baseline value by time-point interaction. | | | |

The percent change from baseline in fasting triglycerides at week 12 (Multiple Imputation Followed by Robust Regression - ITT Analysis - ITT Population) results are provided in Table 15, below.

**Table 15**

| Fasting triglycerides | | Placebo (N=24) | Alirocumab 150 Q2W (N=45) |
|---|---|---|---|
| Baseline (mmol/L) | | | |
| | Combined estimate for mean (SE) | 1.263 (0.180) | 1.446 (0.125) |
| | | | |

| Baseline (mg/dL) | | | |
|---|---|---|---|
| | Combined estimate for mean (SE) | 111.7 (15.9) | 128.0 (11.1) |
| | | | |

| Week 12 percent change from baseline (%) | | | |
|---|---|---|---|
| | Combined estimate for adjusted mean (SE) | 3.9 (5.7) | -7.4 (4.2) |
| | Combined estimate for adjusted mean difference (SE) (Alirocumab vs Comparator) | | -11.3 (7.1) |
| | 95% CI | | (-25.2 to 2.6) |
| | p-value vs Placebo | | 0.1112 |
| | | | |
| Note: The two-step multiple imputation procedure is used to address missing values in the randomized population (seeds=1628 and 3256; number of imputations=100 and 1 in the two steps respectively). In the first step, the monotone missing pattern is induced in the multiply-imputed data. In the second step, the missing data at subsequent visits are imputed using the regression method for continuous variables. | | | |
| Combined estimates and standard errors (SE) are obtained by combining adjusted means and SE from robust regression model analyses of the different imputed data sets. The robust regression models include the fixed categorical effect of treatment group and randomization strata as per IVRS and the continuous fixed covariate of baseline TG value. Rubin's formulae are used to combine means and SE. | | | |

Alirocumab treatment resulted in reductions in LDL-C in hoFH patients with various genotypes, including homozygous (LDLR), compound heterozygous (LDLR), double heterozygous (LDLR + APOB or PCSK9) and heterozygous (LDLR + other benign variants), with the expected minimal to no effect in null/null patients. No LDL-C reductions were observed in placebo-treated patients with any genotype.

In summary, after a 2-week screening period, 69 patients were randomized to study treatment (24 placebo; 45 alirocumab) with comparable demographics in both groups. At the time of randomization, 97% patients were on high intensity statin; 72% on ezetimibe; 17.4% on apheresis; mean baseline LDL-C was 259.6 mg/dL in placebo; 295.0 mg/dL in alirocumab. Difference in LDL-C change from baseline at week 12 was -35.6% (alirocumab [-26.9%] vs. placebo [8.6%]; P<0.0001). Secondary endpoints: ApoB -29.8% (P<0.0001); non-HDL-C -32.9% (P<0.0001); total cholesterol -26.5% (P<0.0001). No treatment-emergent SAEs occurred; no discontinuation due to a TEAE; no deaths reported.

Thus, treatment with alirocumab resulted in statistically significant and clinically meaningful reductions in LDL-C in patients with hoFH, observed early at visit week 4, and subsequently maintained throughout the 12-week double-blind treatment period. Treatment with alirocumab also resulted in significant reductions in other lipoprotein and lipid measures associated with elevated cardiovascular risk (Apo B, total cholesterol, non-HDL-C and Lp(a)). Finally, alirocumab was generally well tolerated with no clinically significant differences between treatment groups with regards to TEAEs, AESIs (adverse events of special interest), and laboratory parameters. Additionally, no safety concerns were identified from the open label data.

## Claims

1. A human antibody that specifically binds human proprotein convertase subtilisin/kexin type 9 (hPCSK9) and comprises a heavy chain comprising SEQ ID NO:5 and a light chain comprising SEQ ID NO:9, a heavy chain variable region (HCVR) comprising SEQ ID NO:1 and a light chain variable region (LCVR) comprising SEQ ID NO:6, wherein the three heavy chain CDRs are set forth in SEQ ID NOs: 2, 3 and 4, and the three light chain CDRs are set forth in SEQ ID NOs: 7, 8 and 10, for use in treating homozygous familial hypercholesterolemia (hoFH) in a patient,
wherein the patient is refractory to treatment with statins, is intolerant to statins, or has a history of adverse reactions to statin therapy; and
wherein the antibody is administered subcutaneously to the patient at a dose of about 150 mg at a frequency of once every two weeks.

2. The human antibody for use according to claim 1, wherein the patient is diagnosed with hoFH based on genotype or clinical criteria.

3. The human antibody for use according to claim 2, wherein the genotype is selected from the group consisting of:
(a) homozygous for mutations in LDLR genes;
(b) compound heterozygous for mutations in LDLR genes; and
(c) double heterozygous for mutations in LDLR, ApoB, or PCSK9 genes.

4. The human antibody for use according to claim 2, wherein the clinical criteria is selected from the group consisting of:
(a) untreated total cholesterol >500 mg/dL (12.93 mmol/L) and triglycerides <300 mg/dL (3.39 mmol/L),
(b) both parents with history of total cholesterol >250 mg/dL (6.46 mmol/L), and
(c) cutaneous or tendinous xanthoma before age 10.

5. The human antibody for use according to any one of claims 1-4, wherein the patient is receiving at least one lipid-modifying therapy (LMT) prior to or at the time of administration of the antibody.

6. The human antibody for use of claim 5, wherein the at least one LMT is LDL apheresis.

7. The human antibody for use according to any one of claims 1-6, wherein the patient has at least about 100 mg/dL LDL-C prior to or at the time of administration of the antibody.

8. The human antibody for use according to any one of claims 1 to 7, wherein the antibody is contained in a pre-filled pen delivery device.

9. The human antibody for use according to any one of claims 1 to 8, wherein the antibody is formulated in a pharmaceutical composition further comprising a pharmaceutically acceptable excipient.

## Patentansprüche

1. Menschlicher Antikörper, der spezifisch an menschliche Proproteinkonvertase-Subtilisin/Kexin vom Typ 9 (hPCSK9) bindet und eine schwere Kette, die SEQ ID NO:5 umfasst, und eine leichte Kette, die SEQ ID NO:9 umfasst, eine variable schwere Kettenregion (HCVR), die SEQ ID NO:1 umfasst, und eine variable leichte Kettenregion (LCVR), die SEQ ID NO:6 umfasst, umfasst, wobei die drei CDRs der schweren Kette unter SEQ ID NO: 2, 3 und 4 dargestellt sind und die drei CDRs der leichten Kette unter SEQ ID NOS: 7, 8 und 10 dargestellt sind, zur Verwendung beim
Behandeln von homozygoter familiärer Hypercholesterinämie (HoFH) bei einem Patienten bzw. einer Patientin,
wobei der Patient bzw. die Patientin gegenüber einer Behandlung mit Statinen refraktär ist, Statine nicht verträgt oder eine Vorgeschichte von negativen Reaktionen gegenüber Statintherapie aufweist; und
wobei der Antikörper dem Patienten bzw. der Patientin in einer Dosis von ungefähr 150 mg in einer Häufigkeit von einmal alle zwei Wochen subkutan verabreicht wird.

2. Menschlicher Antikörper zur Verwendung nach Anspruch 1, wobei der Patient bzw. die Patientin mit hoFH basierend auf Genotyp oder klinischen Kriterien diagnostiziert wird.

3. Menschlicher Antikörper zur Verwendung nach Anspruch 2, wobei der Genotyp aus der Gruppe bestehend aus den Folgenden ausgewählt ist:
(a) homozygot für Mutationen in LDLR-Genen;
(b) heterozygote Verbindung für Mutationen in LDLR-Genen; und
(c) doppelt heterozygot für Mutationen in LDLR-, ApoB- oder PCSK9-Genen.

4. Menschlicher Antikörper zur Verwendung nach Anspruch 2, wobei die klinischen Kriterien aus der Gruppe bestehend aus den Folgenden ausgewählt sind:
(a) unbehandeltem Gesamtcholesterin >500 mg/dl (12,93 mmol/l) und Triglyceride <300 mg/dl (3,39 mmol/l),
(b) beide Eltern mit einem Gesamtcholesterin >250 mg/dl (6,46 mmol/l) in der Vorgeschichte und
(c) kutanem oder tendinösem Xanthom vor einem Alter von 10 Jahren.

5. Menschlicher Antikörper zur Verwendung nach einem der Ansprüche 1-4, wobei der Patient bzw. die Patientin vor oder zum Zeitpunkt der Verabreichung des Antikörpers mindestens eine lipidmodifizierende Therapie (LMT) erhält.

6. Menschlicher Antikörper zur Verwendung nach Anspruch 5, wobei es sich bei der mindestens einen LMT um LDL-Apherese handelt.

7. Menschlicher Antikörper zur Verwendung nach einem der Ansprüche 1-6, wobei der Patient bzw. die Patientin vor oder zum Zeitpunkt der Verabreichung des Antikörpers mindestens etwa 100 mg/dl LDL-C aufweist.

8. Menschlicher Antikörper zur Verwendung nach einem der Ansprüche 1 bis 7, wobei der Antikörper in einer vorgefüllten Stift-Verabreichungsvorrichtung enthalten ist.

9. Menschlicher Antikörper zur Verwendung nach einem der Ansprüche 1 bis 8, wobei der Antikörper in einer pharmazeutischen Zusammensetzung formuliert ist, die ferner einen pharmazeutisch unbedenklichen Exzipienten umfasst.

## Revendications

1. Anticorps humain qui se lie spécifiquement à la proprotéine convertase subtilisine/kexine de type 9 humaine (hPCSK9) et comprend une chaîne lourde comprenant SEQ ID NO:5 et une chaîne légère comprenant SEQ ID NO:9, une région variable de chaîne lourde (HCVR) comprenant SEQ ID NO:1 et une région variable de chaîne légère (LCVR) comprenant SEQ ID NO:6, où les trois CDR de chaîne lourde sont décrites dans SEQ ID NO:2, 3 et 4, et les trois CDR de chaîne légère sont décrites dans SEQ ID NO:7, 8 et 10, pour une utilisation dans un traitement de l'hypercholestérolémie familiale homozygote (hoFH) chez un patient,
dans lequel le patient est réfractaire au traitement par des statines, est intolérant aux statines ou a des antécédents de réactions indésirables à une thérapie par des statines ; et
dans lequel l'anticorps est administré de manière sous-cutanée au patient à une dose d'environ 150 mg à une fréquence de une fois toutes les deux semaines.

2. Anticorps humain pour utilisation selon la revendication 1, dans lequel le patient est diagnostiqué avec hoFH sur la base d'un génotype ou de critères cliniques.

3. Anticorps humain pour utilisation selon la revendication 2, dans lequel le génotype est choisi dans le groupe constitué par :
(a) homozygote pour les mutations dans les gènes LDLR ;
(b) composé hétérozygote pour des mutations dans des gènes LDLR ; et
(c) double hétérozygote pour des mutations dans des gènes LDLR, ApoB, ou PCSK9.

4. Anticorps humain pour utilisation selon la revendication 2, le critère clinique étant choisi dans le groupe constitué par :
(a) cholestérol total non-traité > 500 mg/dL (12,93 mmol/L) et triglycérides < 300 mg/dL (3,39 mmol/L),
(b) les deux parents ont des antécédents de cholestérol total > 250 mg/dL (6,46 mmol/L), et
(c) xanthome cutané ou tendineux avant un âge de 10 ans.

5. Anticorps humain pour utilisation selon l'une quelconque des revendications 1 à 4, dans lequel le patient reçoit au moins une thérapie de modification de lipides (LMT) avant ou au moment de l'administration de l'anticorps.

6. Anticorps humain pour utilisation selon la revendication 5, dans lequel l'au moins une LMT est une aphérèse LDL.

7. Anticorps humain pour utilisation selon l'une quelconque des revendications 1 à 6, dans lequel le patient a au moins environ 100 mg/dL de LDL-C avant ou au moment de l'administration de l'anticorps.

8. Anticorps humain pour utilisation selon l'une quelconque des revendications 1 à 7, dans lequel l'anticorps est contenu dans un dispositif d'administration de type stylo prérempli.

9. Anticorps humain pour utilisation selon l'une quelconque des revendications 1 à 8, dans lequel l'anticorps est formulé dans une composition pharmaceutique comprenant en outre un excipient pharmaceutiquement acceptable.
